# EUROPEAN PATENT SPECIFICATION

(11) **EP 2 935 577 B1**
(45) Date of publication and mention of the grant of the patent: **04.10.2017**
(21) Application number: 13817679.7
(22) Date of filing: 19.12.2013
(51) Int. Cl.: C12N 9/50, C12N 15/62, C12P 21/06

(54) **METHOD FOR PRODUCING A RECOMBINANT PROTEIN OF INTEREST**
VERFAHREN ZUR HERSTELLUNG EINES REKOMBINANTEN PROTEINS VON INTERESSE
PROCÉDÉ DE PRODUCTION D'UNE PROTÉINE RECOMBINANTE D'INTÉRÊT

(30) Priority: 19.12.2012 EP 12198007
(43) Date of publication of application: 28.10.2015
(73) Proprietor: Sandoz AG, 4056 Basel (CH); Boehringer Ingelheim RCV GmbH & Co KG, 1121 Wien (AT)
(72) Inventor: SPONRING, Michael, A-6123 Terfens (AT); SCHNEIDER, Rainer, A-6300 Wörgl (AT); AUER, Bernhard, A-6020 Innsbruck (AT)
(74) Representative: Sonn & Partner Patentanwälte
(86) International application number: PCT/EP2013/077472
(87) International publication number: WO 2014/096245

(56) References cited:
- WO-A1-01/11056
- WO-A2-2006/113957
- CLEMENS ACHMÜLLER ET AL: "N-pro fusion technology to produce proteins with authentic N termini in E-coli", NATURE METHODS, NATURE PUBLISHING GROUP, GB, vol. 4, no. 12, 1 December 2007 (2007-12-01), pages 1037-1043, XP002663733, ISSN: 1548-7091, DOI: 10.1038/NMETH1116 [retrieved on 2007-11-18] cited in the application
- GERRIT VOLKMANN ET AL: "Controllable protein cleavages through intein fragment complementation", PROTEIN SCIENCE, vol. 18, no. 11, 1 November 2009 (2009-11-01), pages 2393-2402, XP055050973, ISSN: 0961-8368, DOI: 10.1002/pro.249 cited in the application
- WALTRAUD KAAR ET AL: "Refolding of N pro fusion proteins", BIOTECHNOLOGY AND BIOENGINEERING, vol. 104, no. 4, 1 November 2009 (2009-11-01), pages 774-784, XP055050976, ISSN: 0006-3592, DOI: 10.1002/bit.22432 cited in the application
- JIANXIN SHI ET AL: "Development of a Tandem Protein Trans-Splicing System Based on Native and Engineered Split Inteins", JOURNAL OF THE AMERICAN CHEMICAL SOCIETY, vol. 127, no. 17, 1 May 2005 (2005-05-01), pages 6198-6206, XP055052473, ISSN: 0002-7863, DOI: 10.1021/ja042287w
- XIAWEI CHENG ET AL: "Expression and purification of antimicrobial peptide CM4 by Nfusion technology in", AMINO ACIDS ; THE FORUM FOR AMINO ACID AND PROTEIN RESEARCH, SPRINGER-VERLAG, VI, vol. 39, no. 5, 29 May 2010 (2010-05-29), pages 1545-1552, XP019856906, ISSN: 1438-2199, DOI: 10.1007/S00726-010-0625-0
- YIFENG LI: "Self-cleaving fusion tags for recombinant protein production", BIOTECHNOLOGY LETTERS, vol. 33, no. 5, 1 May 2011 (2011-05-01), pages 869-881, XP055052800, ISSN: 0141-5492, DOI: 10.1007/s10529-011-0533-8

## Description

The present invention relates to a process for the recombinant production of a desired heterologous polypeptide of interest by using the autoprotease Npro of Pestivirus-technology.

Inteins (INTernal protEINS) constitute protein sequences surrounded by exteins. Inteins catalyse protein splicing without any additional cofactors. In a first step a thioester bond or an ester bond is formed at the N-terminal splicing junction by an acyl shift of a cysteine or serine residue. A transesterification connects the N-terminal extein with the C-terminal extein. Cyclization of an asparagine at the C-terminal splice site results in a free intein and in thio (ester) linked exteins. A second acyl shift yields a natural peptide bond between the N-terminal and C-terminal extein.

Cis splicing as well as trans splicing inteins have been found in all domains of life. Trans acting inteins are split into an N-terminal and a C-terminal fragment. After reassembling of both fragments an active intein is formed. This principle offers many possibilities in biotechnological applications. Inteins can be used for protein purification, protein backbone cyclization, labelling proteins, array construction, and introduction of unnatural substitutions.

One application of inteins is in the field of antimicrobial drug development by inhibition of intein splicing. Inteins are generally found interrupting the catalytic core of essential proteins. However, there are no known inteins in animals or humans. 60 different molecules were identified with low IC50 values, which inhibit Mtu recA and dnaB inteins in an effort to conquer Mycobacterium tuberculosis. However, it has not been proven that the antimicrobial activity is by inhibition of inteins and not by other effects like alkylation of active site cysteins.

Intein based cyclization of therapeutic peptides makes them resistant to exopeptidases and reduces their entropy. Polypeptides can be cyclized in vivo as well as in vitro. The DnaE split intein from Synechocystis sp PCC6803 (Ssp DnaE split intein) was used to cyclize the eight amino acid long tyrosinase inhibitor pseudostellarin F in vivo and by doing so enhancing its production rate. Furthermore, by cyclization of the E. coli dihydrofolate reductase its thermostability could be improved. In vitro experiments used the Ssp DnaE split intein to mediate splicing as well as peptide bond cleavage. In addition trans splicing was accomplished on column using a chitin resin for ligation of two proteins under native conditions. Inhibitors of E. coli Dam methyltransferase were identified using a method named Split Intein Circular Ligation of Peptides and Proteins (SICLOPPS). The IC50 values of the cyclized peptides were much lower than that of the linear ones and in the same range as the naturally occurring inhibitor sinefungin.

Protein purification using inteins is useful because there is no need for a protease to cleave off a fusion protein or an affinity tag. Intein mediated Expressed Protein Ligation (EPL) was successfully used to generate doubly phosphorylated Serotonin N-acetyltransferase (AANAT). This changes its catalytic activity and stability. Furthermore, using this protein semisynthesis method fluorescently labelled 14-3-3ζ was generated which was then used to quantify the binding of mono- and diphosphorylated AANAT by anisotropy. More protein modifications using EPL include introduction of selenocysteine, unnatural amino acids, FRET pairs, biotin or other tags, membrane-protein anchors, DNA, and photo-activated compounds.

The split intein Ssp GyrB S11 can be divided into an extremely small (6 aa) C-intein and a large (150 aa) N-intein. This system can be used for C-terminal labelling of recombinant proteins with modified peptides.

An intein based biosensor for measuring the activity of the caspase-3 was developed using the Ssp DnaE split intein. Firefly luciferase (Luc) was connected with caspase-3 substrates. After cyclization of Luc its function was abolished until a protease generated a linear Luc protein. In Shi et al. (J. Am. Chem. Soc. 127 (17) (2005): 6198-6209), development of a tandem protein trans-splicing system based on native and engineered split inteins is disclosed.

Another application for inteins is in the field of in vivo control of protein function. A thyroid hormone binding domain was inserted into an intein and hence abolishing its splicing activity. By addition of human thyroid hormone or analogues the intein gained its functions back and the active intein was used to activate a variety of different proteins in E. coli in a dose depend manner.

The S. cerevisiae VMA intein (vacuolar ATPase subunit) was used as a temperature sensitive switch to control the activation of the transcription regulators Gal4 and Gal80. The Gal80-intein was able to regulate the Gal4 upstream activation sequence temperature dependent in D. melanogaster.

Trans splicing can also be a valuable tool in gene therapy. Coagulation factor VIII (FVIII) is secreted as a heterodimer consisting of a heavy chain (HC) and a light chain (LC) which is secreted much more efficient than the HC. The results of these trans splicing experiments suggest that the FVIII LCs are critical in maintaining the proper configuration of HCs for secretion.

Trans splicing using the Ssp DnaE intein can be conducted in plants as well. The N-terminal fragments of 5-enol-pyruvylshikimate-3-phosphate synthase (EPSPS) fused to the N-terminal part of Ssp DnaE was integrated into the nuclear DNA of N. tabacum. The remaining EPSPS gene fragment fused to the C-terminal part of Ssp DnaE was integrated into the chloroplast genome. Trans splicing utilized full length EPSPS and improved resistance to the herbicide N-phosphonomethyl-glycine (glyphosphate).

Drawbacks of intein based technologies are spontaneous cleavages during protein expression and purification (Volkmann et al. (Protein Science 18 (2009), 2393-2402). This significantly decreases the yield of the desired protein product and complicates subsequent uses of the cleaved protein. Another issue is the specificity of inteins. A screening using SICLOPSS showed that less than 70% of randomly selected clones showed detectably levels of in vivo cyclization (Cheriyan et al., Adv.Drug Del. Rev. 61 (2009), 899-907).It is an object of the present invention to provide an improved alternative to intein technology for producing recombinant proteins, especially proteins for therapeutic use in humans. The method should be easily controllable and suitable for up-scaling to provide industrial production of such proteins.

Therefore, the present invention provides a method for producing a recombinant protein of interest which is characterised by the following steps:
(a) providing a first part of an N^{pro} autoprotease and providing a second part of an N^{pro} autoprotease, wherein said second part is fused by a peptidic bond to a protein of interest but said second part alone does not exhibit a proteolytic activity, and wherein complementation of said first part with the second part forms an autoproteolytically active N^{pro} autoprotease,
(b) contacting the first part of the N^{pro} autoprotease with the second part of the N^{pro} autoprotease so that an autoproteolytically active N^{pro} autoprotease is formed and the protein of interest fused by the peptidic bond to the second part of the N^{pro} autoprotease is proteolytically cleaved off the second part of the N^{pro} autoprotease at the peptidic bond, and
(c) recovering the protein of interest,
wherein the first part of the N^{pro} autoprotease comprises amino acid 22 to 30 of N^{pro} autoprotease and that the second part of the N^{pro} autoprotease comprises amino acid 113 to 168 of N^{pro} autoprotease.

The present invention is an improvement in the recombinant production of a desired heterologous polypeptide of interest by using the autoprotease N^{pro} of Pestivirus-technology. This technology usually provides the recombinant expression of a fusion polypeptide which comprises an autoproteolytic moiety directly or indirectly derived from autoprotease N^{pro} of Pestivirus and a heterologous polypeptide of interest in a host cell, often a prokaryotic host cell, such as E. coli. The heterologous polypeptide or protein of interest is covalently coupled via a peptide bond to the N^{pro} molecule. The protein of interest is released from the fusion protein through hydrolysis of the peptide bond between the C-terminal Cys168 of Npro and position 169 of the fusion polypeptide which represents the authentic N-terminal amino acid of the protein of interest to be produced according to the present invention. The heterologous polypeptide of interest is produced in the host cell in form of cytoplasmic inclusion bodies (IB), which are then isolated and treated in such a way, that the desired heterologous polypeptide is cleaved from the fusion polypeptide by the N^{pro} autoproteolytic activity. With the present invention, the metabolic burden of the host cell can be significantly reduced because the first part of the N^{pro} autoprotease does not have to be expressed together with the fusion polypeptide comprising the protein of interest (it can be produced separately). This leads to significantly higher yields and shorter production times.

Fusion polypeptides comprising the autoprotease Npro of Pestivirus are therefore specifically useful for producing heterologous recombinant polypeptides. N^{pro} is an autoprotease with length of 168 amino acids and an apparent Mᵣ of about 20 kD in vivo. It is the first protein in the polyprotein of Pestiviruses and undergoes autoproteolytic cleavage from the following nucleocapsid protein C. This cleavage takes place after the last amino acid in the sequence of N^{pro}, Cys168. The autoprotease N^{pro} activity of Pestivirus always cleaves off the fusion partner at this clearly determined site, releasing a polypeptide of interest with homogenous N-terminus. In addition, the autoproteolytic activity of N^{pro} can be induced in vitro, by application of special buffers, so that the polypeptide of interest can be obtained by cleavage of fusion polypeptides that are expressed in IBs.

N-terminal autoprotease N^{pro} from Classical Swine Fever Virus (CSFV) used in this technology serves as an attractive tool for expression of therapeutic proteins in large amounts especially in E. coli. Medical applications require an authentic N-terminus of the recombinant proteins, which can be achieved by self-cleavage of N-terminally fused N^{pro} autoprotease. In addition, N^{pro} fusion technology also allows the expression of small or toxic peptides, which would be degraded immediately after their synthesis by host cell proteases (Achmüller et al., Nat. Methods 4 (2007), 1037-1043). As the expression of N^{pro} fusion proteins in E. coli leads to the formation of insoluble aggregates, known as inclusion bodies, appropriate resolving and renaturation protocols are required to obtain biological active proteins.

As already mentioned, in most cases solubilisation is carried out in chaotropic agents such as urea or guanidinium chloride at high concentrations in combination with reducing agents to abolish false formed disulfide bonds. Due to its simplicity refolding by dilution is widely used to initiate renaturation.

The present invention is carried out with the N^{pro} technology. This technology is disclosed e.g. in WO 01/11057 A, WO 01/11056 A, WO 2006/113957 A, WO 2006/113958 A, WO 2006/113959 A, Cheng et al., Amino Acids 39 (5) (2010): 1545-1552; and Achmüller et al., Nat. Meth. 4 (2007), 1037-1043, and reviewed e.g. in Li, Biotechnol. Lett. 33 (2011), 869-881. In general terms, the N^{pro} technology relates to a process for the recombinant production of a heterologous protein of interest, comprising (i) cultivation of a bacterial host cell which is transformed with an expression vector which comprises a nucleic acid molecule which codes for a fusion protein, the fusion protein comprising a first polypeptide which exhibits the autoproteolytic function of an autoprotease N^{pro} of a Pestivirus, and a second polypeptide which is connected to the first polypeptide at the C-terminus of the first polypeptide in a manner such that the second polypeptide is capable of being cleaved from the fusion protein by the autoproteolytic activity of the first polypeptide, and the second polypeptide being a heterologous protein of interest, wherein cultivation occurs under conditions which cause expression of the fusion protein and formation of corresponding cytoplasmic inclusion bodies, (ii) isolation of the inclusion bodies from the host cell, (iii) solubilisation of the isolated inclusion bodies, (iv) dilution of the solubilisate to give a reaction solution in which the autoproteolytic cleavage of the heterologous protein of interest from the fusion protein is performed (the present invention specifically aims on this step by providing an alternative embodiment to this step), and (v) isolation of the cleaved heterologous protein of interest ("recovering"). According to the present invention, the first polypeptide, the N^{pro} autoprotease, is provided in a cleaved status, the method according to the present invention establishing (auto)proteolytic activity by complementation of the first and second part of the first polypeptide (therefore, the "auto"proteolytic activity is an activity of the re-combined N^{pro} molecule).

The term "denatured form" (or "non-refolded form") in the meaning of the present invention designates the biologically inactive form of the expressed fusion protein comprising the second part of the N^{pro} autoprotease as well as the biologically inactive form of the first part of the N^{pro} autoprotease, as obtained as a product of the recombinant production process, usually as obtained after solubilising the inclusion bodies (under conditions under which the native three dimensional structure of the fusion protein (as well as of the first part of the N^{pro} autoprotease) is disrupted).

The term "refolding" (or "renaturing") refers to the mechanism during which the solubilized protein or part of protein regains its native conformation and biological activity (or the biological activity of the part, respectively), i.e. reconstituting a protein from its denatured, inactive state to its active form (see also: Kaar et al., Biotech. Bioeng. 104 (2009), 774-784).

The term "autoproteolytic function", "autoproteolytic cleavage" or "autoproteolytic" refers to the proteolytic activity of the complementation of the first part with the second part of the autoprotease, wherein the second part is fused to the protein of interest (insofar, the term "autoprotease" according to the present invention is used in a different way as usual, because the proteolytic activity is not drawn to the protein having the proteolytic activity, but drawn to the complementation of the first part of the N^{pro} autoprotease and the second part of the N^{pro} autoprotease (the latter including the protein of interest); however, the two parts were generated from a "classical" autoprotease). As used herein the term "autoprotease" shall therefore refer to the complemented first and second part of a polypeptide that possesses proteolytic activity and is capable of cleaving the other (second) part of the autoprotease from the protein of interest, i.e. the complemented N^{pro} autoprotease cleaves (via its proteolytic activity generated by the complementation step) its second part from the fusion protein (the fusion protein comprising the second part of the autoprotease fused to the protein of interest). The first part of the N^{pro} autoprotease may have already proteolytic activity; the fusion protein comprising the second part of the N^{pro} autoprotease must not have autoproteolytic activity (so that cleavage of the protein of interest from the second part of the Npro autoprotease cannot occur until complementation with the first part of the N^{pro} autoprotease). Accordingly, complementation of said first part with the second part can be effected to provide an autoproteolytically active N^{pro} autoprotease. This complementation can be directed by appropriate renaturing conditions which do not only allow spatial coordination of the two parts of the N^{pro} autoprotease, but also functional restoration of the autoproteolytic activity for effecting the cleavage event.

This cleavage event can be directed by various embodiments according to the present invention. There are also various possibilities for providing the first part of the N^{pro} autoprotease and the fusion protein comprising the second part of the N^{pro} autoprotease. For example, the first part of the N^{pro} autoprotease can be expressed/produced by fermentation solely. Alternatively or additionally, the second part of the N^{pro} autoprotease (as fusion protein) can be expressed/produced by fermentation solely. Further embodiments include the possibility to provide the first part of the N^{pro} autoprotease and/or the second of the N^{pro} autoprotease (as fusion protein) as inclusion bodies (IB); the two polypeptides (or only one of them) can be provided as soluble forms in cytoplasm (preferably the shorter polypeptide (usually the first part). Also combinations are possible (e.g. to provide the first part soluble and second part as IB (or the first part as IB and second soluble). The cleavage event can be diligently directed within the rationale of the present invention. For example, a denaturated first part can be mixed with a denaturated second part, refolded together and effect cleavage; the first part may also be refolded separately from the second part, mixed with the (also separately refolded) second part and allowed to cleave. It is also possible, e.g. to refold the first part, mix it with the denatured second part, refold the second part and effect cleavage. Another preferred embodiment comprises the provision of the first part in denaturated form and the second part in refolded form, mix the polypeptides, refold the first part and allow cleavage.

The autoproteolytic cleavage rate can be determined, for example, by initially solubilising the isolated/purified inclusion bodies (with the expressed fusion protein comprising the second part of the autoprotease fused to the protein of interest) in 7 M guanidine/HCI solution and then diluting 1:100 in reaction solution and adding the first part of the autoprotease to initiate proteolytic activity of the complemented autoprotease. After incubation for about 24 h, the reaction solution is examined by SDS-PAGE for cleavage (of the second part of the autoprotease from the protein of interest) having taken place. A Western blot is carried out to identify the proportions of processed (the second part of the N^{pro} autoprotease has been cleaved off from the fusion protein thereby liberating the protein of interest) and unprocessed (the second part of the N^{pro} autoprotease is still fused to the protein of interest) fusion protein. The proportion of cleaved material can be determined by densitometric analysis of the Coomassie-stained SDS-PAGE gel.

As used herein the term "inclusion bodies" shall refer to insoluble aggregates containing heterologous polypeptides (the fusion protein according to the present invention comprising the second part of the autoprotease fused to the protein of interest; of course, also the first part of the autoprotease can be expressed in inclusion bodies) present in the cytoplasm of transformed host cells. These appear as bright spots under the microscope and can be recovered by separation of the cytoplasm. The inclusion bodies are usually solubilised using a chaotropic agent. Upon solubilisation inclusion bodies are dissolved and a monomolecular suspension with substantially reduced intra- and inter-molecular interactions is aimed to obtain. Preferred solvents are urea, guanidine HCl and strong ionic detergents as N-lauroylsarcosine. In another embodiment of the present invention inclusion bodies are also solubilised using an aqueous alcohol solution at alkaline pH or simply an aqueous solution at alkaline pH.

As used herein, the term "recovering" shall refer to the obtaining of the protein of interest in purified form, i.e. by essentially separating the protein of interest from other proteins present in the expression/processing system, especially any (fragments) of the autoprotease, but also all other proteins or other components which should not be present in the intermediate or final product. The protein of interest recovered by the method according to the present invention may be commercialised in the bulk form obtained directly from the cleaving step according to the present invention or further purified and/or formulated into a specific formulation, e.g. into a pharmaceutical formulation.

As used herein the term "solubilisation" shall refer to the process necessary to dissolve the inclusion bodies. Solubilisation is aimed to result in a monomolecular dispersion of the polypeptides with minimum intra- and inter-molecular interactions. A preferred way of solubilisation of inclusion bodies within the scope of the present invention, is conducted by suspension in 50 mM Tris/HCl, 8 M urea, pH 7.3, adding a reducing agent, e.g. 50 mM DTT, in the case that oxidized cysteine residues are present. Where necessary it is possible to remove potentially insoluble material, for example by centrifugation. In the case that the inactive fusion protein is produced soluble within the cell, the clarified cell homogenate is subjected to the further work up described below for the solubilized inclusion bodies.

This technology is suited for a large variety of proteins of interest. For the purpose of the present invention, the terms "heterologous protein", "target protein", "polypeptide of interest" or "protein of interest" (and the like) mean a polypeptide which is not naturally cleaved by an autoprotease N^{pro} of a Pestivirus from a naturally occurring fusion protein or polyprotein (i.e. a polypeptide being different than the naturally following amino acids 169ff of the Pestivirus polyprotein encoding the structural Protein C and subsequent viral proteins). Examples of such heterologous proteins of interest are industrial enzymes (process enzymes) or polypeptides with pharmaceutical, in particular human pharmaceutical, activity.

Examples of preferred proteins of interest with human pharmaceutical activity are cytokines such as interleukins, for example IL-6, interferons such as leukocyte interferons, for example interferon a2B, growth factors, in particular haemopoietic or wound-healing growth factors, such as G-CSF, erythropoietin, or IGF, hormones such as human growth hormone (hGH), antibodies or vaccines. Also very short polypeptides having only 5 to 30 amino acid residues can be produced as protein of interest by the present technology. The N^{pro} technology has specific advantages in an expression system making use of inclusion bodies, because the strong aggregation bias of the fused autoprotease facilitates the formation of inert inclusion bodies, almost independent of the fusion partner. Accordingly, almost any protein of interest is producible with the present system in high amounts and yields. Reports are e.g. available for expression of synthetic interferon-a1; toxic gyrase inhibitor CcdB, a short 16-residue model peptide termed pep6His (SVDKLAAALEHHHHHH (SEQ.ID.N0.12)), human proinsulin, synthetic double domain D of staphylococcal protein A (sSpA-D₂), keratin-associated protein 10-4 (KRTAP10-4), synthetic green fluorescent protein variant (sGFPmut3.1), synthetic inhibitorial peptide of senescence evasion factor with N-terminal cysteine (C-sSNEVi), synthetic inhibitorial peptide of senescence evasion factor with randomized amino acid sequence with C-terminal cysteine (sSNEVscr-C); recombinant human monocyte chemoattractant protein 1 (rhMCP-1). So far, the only limitations with respect to high yields have been suspected for chaperones and proteins with comparable properties of supporting protein folding. Such proteins as fusion partners could suppress the aggregation bias of an N^{pro} molecule, leading to lower yields due to less aggregation. Nevertheless, the present technology can even be applied for expressing such proteins counter-acting aggregation.

The fusion protein according to the present invention can additionally contain auxiliary sequences, such as affinity tags or refolding aid moieties; it may also contain more than one protein of interest (it can e.g. contain two or three or four or even more proteins of interest which may be separated from each other at a later stage or even at the same stage as the cleavage by the Npro autoprotease).

With the present invention, an improvement is introduced into N^{pro} technology insofar that not the complete fusion protein consisting of N^{pro} autoprotease fused to a protein of interest has to be expressed in the expression system for producing the protein of interest, but that the first part of the N^{pro} moiety exhibiting the proteolytic activity is produced (e.g. expressed) separately so that autoproteolytic function can be selectively initiated by combining the first part with the second part of the autoprotease (the latter being fused to the protein of interest). Since the fusion protein according to the present invention (comprising the second part of N^{pro} autoprotease and the protein of interest) is smaller than the fusion protein in classical N^{pro} autoprotease technology (wherein the whole N^{pro} autoprotease (including the first part) is expressed), and therefore easier to express in recombinant production systems and easier to handle in processing before the cleaving step, the present invention allows higher titers and better productivity with respect to the protein of interest. Combination of the first (proteolytic) part and the second part (with the protein of interest) leads to complementation of the N^{pro} autoprotease, whereafter the autoproteolytic activity can act for cleaving off the protein of interest from the second part of the N^{pro} molecule.

As already mentioned, N-terminal autoprotease N^{pro} from Classical Swine Fever Virus (CSFV) can be used as an excellent tool for expression of recombinant proteins, especially in E. coli. Due to its autocatalytic cleavage it enables synthesis of proteins with an authentic N-terminus which is especially important for pharmaceutical applications. Furthermore, not only large proteins ("proteins of interest") but also small peptides can be stably expressed by C-terminal linking to N^{pro}. A high expression rate forces the fusion protein into inclusion bodies. After purification, N^{pro} is refolded and cleaves itself off after complementation according to the present invention.

N^{pro} is advantageous compared to intein technologies especially concerning its fusion partners, which can be cleaved off. Similar to split inteins, N^{pro} can be split up into two fragments, for example one ("first") ranging from amino acid 22-106 and the other ("second") part ranging from amino acid 113-168. It is essential that the second (C-terminal) part alone does not already lead to autoproteolytic cleavage of the fusion protein, but that cleavage ("liberation" of the protein of interest only occurs upon effective complementation with the first part of the N^{pro} autoprotease. By adding the first (N-terminal) part of N^{pro} a functional protease is formed by complementation and the fusion partner is cleaved off.

It is essential that the protein of interest to be produced by the present invention is attached C-terminally after Cys168 of the N^{pro} autoprotease, because this is the cleavage site where the peptidic bond between the C-terminus of the N^{pro} moiety (at Cys168) and the protein of interest is cleaved upon complementation in step (b) according to the present invention.

In comparison with the intein system, the present N^{pro} complementation system according to the present invention is superior over intein technologies. A major advantage over the intein-system is, that with the split Npro technique it is possible to produce peptides/proteins with authentic homogeneous N-termini (meaning a protein product in which all molecules have the same N-terminus; standard protein production in E. coli can lead to a mixture of fMet, Met, and correct N-terminus which is especially important for biopharmaceuticals to guarantee the authentic activity and to avoid adverse drug reactions elicited by non-natural N-termini. Especially if one N^{pro} fragment is bound to a solid support and the other part of N^{pro} is added, the proteolytic activity is triggered by the complementation on the solid support and the fusion protein is cleaved off. The division of N^{pro} into two fragments offers many applications in addition to a complementation system. The site at which Npro is subdivided can be used to integrate new features, such as an affinity tag. For example, 6 consecutive His residues can be introduced at positions 107 to 112 instead of the original sequence, thereby creating a His-tag. The resulting N^{pro} autoprotease still has proteolytic activity but the His-tag can be used for affinity chromatography. As another example, chaperone-like structures can be introduced into the autoprotease molecule, e.g. SlyD. SlyD is a member of the FKBP family and fulfils many duties in E. coli. The flap (IF) domain of SlyD has a chaperone-like activity with a high affinity to unfolded proteins. SlyD also contains a C-terminal stretch with many His residues which serves as a nickel storage and nickel supplier for the formation of Ni-Fe clusters. This nickel ion binding also regulates peptidyl prolyl isomerase (PPI) activity, which is responsible for accelerating the rate limiting trans to cis isomeration in protein folding. N^{pro} contains many Pro residues which could impair refolding. SlyD improves the folding of Npro in combination with its PPI activity. The nickel binding site can be used as an affinity tag for purification. The IF domain can ideally be integrated between the N-terminal and C-terminal fragment of N^{pro} due to the near vicinity to its N- and C-terminus. This adds a chaperone activity to N^{pro}.

Accordingly, in a preferred embodiment of the present method, the first and/or second part of the N^{pro} autoprotease were generated in a recombinant production system, preferably in a prokaryotic host cell, especially in E. coli host cells. Preferably, both, the first and the second part are generated in a recombinant production system. This can be performed in one or two production systems: If produced in one production systems, both, the first and the second part are generated in the same recombinant production system. Producing the parts in separate recombinant production systems may be advantageous with respect to flexibility; production in the same recombinant system may be advantageous with respect to production facilities. It is also possible to produce both parts in inclusion bodies or only one of them or none. Refolding can then be adapted in a flexible manner, due to the systems.

According to a preferred embodiment, at least the second part (including the protein of interest) is generated as inclusion bodies, then solubilised and brought into contact with the first part (in step (b) according to the present invention). The first part can also be generated in inclusion bodies and may or may not be allowed to refold before contact with the second part. In this preferred embodiment, refolding of the second part can e.g. be achieved simultaneously in step (b) of the present invention.

In general, the sequence of providing the two polypeptides according to the present invention as inclusion bodies, solubilising, refolding and performing step (b) of the present invention may, however, be adapted to the nature and physicochemical behaviour of the polypeptides in each individual case. For example, the polypeptides may be mixed already at an early stage (or even be present in the same expression system as inclusion body), or not contacted with each other until performance of step (b) according to the present invention. It follows e.g. that the steps of solubilising or refolding can be performed with the polypeptides mixed with each other or being conducted separately; also conditions can be applied which allow solubilisation/refolding of only one of the two parts (even being already mixed) where the other part does not (yet) solubilise/refold. Step (b) may then be initiated by applying suitable conditions which allow complementation and cleavage to cleave the protein of interest from the second part of the N^{pro} autoprotease.

Preferred examples for the first part of the N^{pro} autoprotease are deletion mutants of naturally occurring versions of the N^{pro} autoprotease. Such deletions, of corse, must not lead to inactivation of proteolytic activity. For example, amino acids 2 to 21 (the amino acid numbering follows the numbering of most naturally occurring N^{pro} autoprotease sequences of CSFV, such as listed in Becher et al., J. Gen. Virol. 78 (1997), 1357-1366) can be deleted without affecting proteolytic activity. It is therefore preferred to use a first part of the N^{pro} autoprotease lacking amino acids 2 to 21 ("Δ21 N^{pro}). These preferred first fragments with proteolytic activity therefore start with the Met + GluPro motif (at positions 1 and 22/23), preferably followed by a (Val/Leu)(Tyr/Phe) motif (amino acids 24 and 25 of N^{pro}). Another example for possible deletion without affecting proteolytic activity is amino acids 148 to 150 (e.g. ThrProArg in "EDDIE" (Achmüller et al., 2007) or GluProArg in the Alfort sequence (Becher et al., 1997)).

According to the present invention, further deletions are possible, even if the proteolytic activity is slightly reduced, e.g. amino acids 26 to 30, 76/77, 108 to 110, 147 and 151.

However, significant reduction in proteolytic activity has to be prevented. Therefore, preferably, the following amino acids should not be deleted (in the first/second part):
- the ProSerGlu motif at amino acids 35 to 37 (also present in variations, such as ProSerAsp, ProSerPro, ProHisPro, etc.);
- the LeuProHis motif (amino acids 47 to 49 of N^{pro}), optionally with the following amino acid (amino acids 50);
- amino acids 54 to 60 (although here, sequence variations may lead to improved proteolytic activity (see WO 2006/113957 A; Achmüller et al., 2007));
- amino acids 83 to 91 and 94/95; and
- amino acids 158 to 160.

Sequence variations occur between the various natural isolates (see e.g. GenBank or EMBL databases); also selected mutations have been provided with improved properties (see WO 2006/113957 A; Achmüller et al., 2007; Achmüller, PhD thesis, March 2006, University of Innsbruck (AT)): For example,
- Cys112, Cys134 and Cys138 can be replaced by another amino acid residue, preferably Glu;
- His5, Lys16, Asn35, Arg53, Gly54, Arg57, Leu143, Lys145 and/or Arg150 can be replaced by another amino acid residue, preferably arginine (R) 53 with glutamic acid (E), glycine (G) 54 with aspartic acid (D), arginine (R) 57 with glutamic acid (E), and/or leucine (L) 143 with glutamine (Q);
- Va124, A1a27, Leu32, Gly54, Leu75, Ala109, Val114, Val121, Leu143, Ile155 and/or Phe158 can be replaced by another amino acid residue, preferably threonine or serine, especially alanine (A) 109, valine (V) 114, isoleucine (I) 155 and/or phenylalanine (F)158;
- Ala28, Ser71 and/or Arg150 can be replaced by another amino acid residue, preferably glutamic acid (E), phenylalanine (F) and/or with histidine (H), especially alanine (A) 28 can be replaced with glutamic acid (E), serine (S) 71 can be replaced with phenylalanine (F) and arginine (R) 150 can be replaced with histidine (H).

A preferred embodiment is the N-terminal protease of Pestivirus strain D32/00_HoBi (GenBank Accession No. AAS68353.1) or the variant Δ21N^{pro} (HoBi) (SEQ.ID.NO.13) thereof having a deletion of the amino acids 2 to 21 at the N-terminus and the "TSC" motif at the C-terminus instead of the naturally occurring "ASC" motif.

According to another aspect, the present invention also relates to an N^{pro} autoprotease moiety (in isolated form or in form of a fusion polypeptide together with a protein of interest or in form of a fragment of a fusion protein that can be complemented according to the present invention, i.e. a C-terminal part of the autoprotease N^{pro} moiety) that has improved chaotropic properties compared to wild type N^{pro} or other known N^{pro} variants, such as the EDDIE variants according to WO 2006/113957 A. The N^{pro} moieties provided with the present invention have an unusual high propensity for beta aggregation. This has been found in the "HoBi" form (UniProt Database accession no. Q5L4B1). This high propensity for beta aggregation correlates with the specific properties of these autoproteases, namely high productivity and renaturation at high chaotropic conditions. With the present invention hydrophobic patches in naturally occuring N^{pro} variants that are compatible with enzymatic activity are provided. Combining such hydrophobic patches on the original HoBi scaffold provided novel active N^{pro} variants with improved properties concerning beta-aggregation during expression, inclusion bodies formation and renaturation. These Npro variants are therefore specifically suited for recombinant expression. Following amino acid exchanges in the HoBi protein sequence (SEQ.ID.No.21), alone or in combinations thereof, are provided in the novel molecules according to the present invention:
N28 to V, L or I; A30 to T; T39 to V or I; L81 to V, F82 to Y, V83 to I, K84 to E, P85 to L, P87 to A, V88 to I, Q91 to K; S94 to I, L or V; Q105 to L; P110 to V; N127 to K, M131 to I, T135 to V, V141 to L. In preferred embodiments of the Npro variants according to the present invention, at least two of such exchanges are present, more preferred, at least three exchanges, especially at least four exchanges.

These amino acid exchanges significantly affect the beta-aggregation propensity of the optimized HoBi-Npro and all these exchanges were tolerated during the evolution of the Pesti-virus species, as these exchanges have been found in enzymatically active Npro-variants.

Therefore, the present invention provides an N^{pro} autoprotease moiety having a sequence comprising at least one amino exchange compared to SEQ ID No. 21, wherein the amino acid exchange is selected from the group consisting of N28 to V, L or I; A30 to T; T39 to V or I; L81 to V, F82 to Y, V83 to I, K84 to E, P85 to L, P87 to A, V88 to I, Q91 to K; S94 to I, L or V; Q105 to L; P110 to V; N127 to K, M131 to I, T135 to V, V141 to L.

The N^{pro} autoprotease moiety according to the present invention may comprise at least two, preferably at least three, especially at least four amino acid exchanges selected from the group consisting of N28 to V, L or I; A30 to T; T39 to V or I; L81 to V, F82 to Y, V83 to I, K84 to E, P85 to L, P87 to A, V88 to I, Q91 to K; S94 to I, L or V; Q105 to L; P110 to V; N127 to K, M131 to I, T135 to V, V141 to L.

Preferably, amino acids 2 to 21 of SEQ ID No. 21 are deleted. This allows even more improved results in protein expression systems.

Another preferred exchange in the N^{pro} autoprotease moieties according to the present invention is the exchange of A166 of SEQ ID No. 21 with T.

The present N^{pro} autoprotease moieties (including the N^{pro} autoprotease moiety according to SEQ ID No. 21) are specifically useful for the recombinant expression of proteins.

The present invention also relates to fusion proteins comprising a recombinant protein of interest and an N^{pro} autoprotease moiety according to the present invention (including the N^{pro} autoprotease moiety according to SEQ ID No. 21). The present invention also relates to nucleic acid molecules comprising a sequence encoding such N^{pro} autoprotease moieties or fusion proteins, especially expression vectors for recombinant expression (with e.g. suitable promoters, marker genes (resistance markers, etc), etc.).

In line with the above teaching, the present invention specifically provides an N^{pro} autoprotease moiety having a sequence selected from the group consisting of SEQ ID nos. 13 or 21 (Δ21N^{pro} (HoBi) or N^{pro} (HoBi)). On the other hand, also known N^{pro} autoprotease sequences can be applied, such as the N-terminal protease of Pestivirus strain D32/00_HoBi (GenBank Accession No. AAS68353.1).

According to a preferred embodiment, the first part of the N^{pro} autoprotease comprises amino acid 22 to 75, more preferred amino acid 22 to 112, 22 to 106, 1 to 106, 1 to 75 or 1 to 30, especially amino acid 1 to 112.

According to a preferred embodiment, the second part of the N^{pro} autoprotease comprises amino acid 31 to 168 of N^{pro} autoprotease, preferably amino acid 76 to 168, especially amino acid 107 to 168.

As already mentioned, the exact sequence and very nature of the autoprotease which is split and complemented in the method according to the present invention is not critical; accordingly N^{pro} autoproteases already successfully used in the N^{pro} autoprotease technology represent preferred embodiments of the present invention. Preferred autoproteases can be chosen also according to the fusion partner ("protein of interest"). For example, preferred sequences are the N^{pro} sequences disclosed in WO 2006/113957 A (as SEQ.ID.NOs. 1-5, 32/33, 92-98, especially SEQ.ID.NO 5 ("EDDIE")), the HoBi sequence (SEQ.ID.Nos. 13 and 21) and the variants disclosed above.

As already stated above, preferred cleavage sites in the N^{pro} sequence of the complement system according to the present invention (that divides the first part of the N^{pro} autoprotease with the second part of the N^{pro} autoprotease (the latter being fused to the protein of interest)) is preferably located from amino acid 30 onwards in the direction of the C-terminus.

Cleavage sites enabling complementation can also be scanned starting from the N-terminus based on the structure information present for N^{pro} (Zögg et al., Structure. 2013; 21 (6) : 929-38), Yu and Lutz, Trends Biotechnol. 2011; 29(1): 18-25; Boissinot et al., EMBO J. 1997; 16(9): 2171-8). A preferred complementing cleavage site can be provided beyond the border (directly following a short alpha-helix) between the catalytic domain of HoBi-N ^{pro} and the protein interaction hub. As the protein interaction hub is not involved in the catalytic process and only the penultimate 8 C-terminal amino acids are again directly engaged in the substrate-recognition/cleavage-reaction, there are several other preferred cleavage sites within the protein interaction hub beyond the experimentally determined one (up to amino acid position 160) enabling a specifically high-yield complementation of active N^{pro}. From circular permutation studies of proteins it is known that loops between secondary structural motifs like e.g. the loop amino acids 144-149 between the two antiparallel beta-sheets in the interaction hub of (HoBi-)N^{pro} are good candidates for the introduction of new N- and C-termini without disturbing the fold of the protein and could thus also serve to cleave the protein to obtain two complementing fragments.

Accordingly, preferred cleavage sites dividing the first part of the N^{pro} autoprotease from the second part of the N^{pro} autoprotease are from amino acid residue 30 to 160, more preferred from amino acid residue 65 to 85, 105 to 120, 140 to 160, especially from 107 to 115 and from 140 to 154 of an N^{pro} autoprotease sequence.

The complementation step (b) can be carried out in solution; however, according to a preferred embodiment, either the first or the second part can be immobilised on a solid support before complementation. Complementation can then be initiated by adding the other part whereafter the proteolytic activity is activated by complementation on the solid support. The cleaved part (either the C-terminal (i.e. the second part of the) autoprotease (if the protein of interest moiety is coupled to the solid support) or the protein of interest (if an autoprotease moiety is coupled to the solid support)) is then released from the solid support and can be easily recovered. Therefore, according to a preferred embodiment of the present method, the first or the second part of the N^{pro} autoprotease (the latter being coupled to the protein of interest) is provided in immobilised form on a solid support and contacted with the second or first part of the N^{pro} autoprotease in step (b) so that the autoproteolytically active N^{pro} autoprotease is formed on the solid support. Immobilisation to the solid support is preferably effected by adsorption or covalent binding.

As solid phase material, all materials already applied in the present field are appropriate. Preferably, the solid phase is selected from the group consisting of chromatography material, especially supports based on cellulose, agarose, acrylamide, poly(styrene-divinylbenzene) or ethylene glycol-methacrylate copolymers, microtiter plates, nitrocellulose membranes, microchips, glass plates, or metal coated supports.

According to the present invention various types of solid phase supports may be used, such as the supports based on cellulose, agarose (Sepharose or Macro-Prep gels), dextran (Sephadex gels), acrylamide (Sephacryl, Trisacryl gels), silica (TSK, SW gels), poly(styrene-divinylbenzene) (Source or Poros gels), ethylene glycol-methacrylate copolymers (Toyopearl HW, TSK, PW, fractogel EMD gels) or mixtures, in particular of agarose and dextran (Superdex gel). The supports approved for human or veterinary use by the competent American authorities (FDA for food and drug administration) or the European Union agencies will be more particularly selected. In addition, the support selected can be bonded, preferably by covalent bonding, to an affinity ligand (the support is said to be functionalized). The solid phase matrix may comprise, as the matrix backbone, any natural or synthetic and organic or inorganic material known per se to be applicable in solid phase separation of proteins and other biomolecules, e.g. natural or synthetic polysaccharides such as agar-agar and agaroses; celluloses, cellulose ethers such as hydroxypropyl cellulose, carboxymethyl celluose; starches; gums such as guar gum, and gum arabic, gum ghatti, gum tragacanth, locust bean gum, xanthan gum; pectins; mucins; dextrans; chitins; chitosans; alginates; carrageenans; heparins; gelatins; synthetic polymers such as polyamides such as polyacrylamides and polymethacrylamides; polyimides; polyesters; polyethers; polymeric vinyl compounds such as polyvinylalcohols and polystyrenes; polyalkenes; inorganic materials such as silicious materials such as silicon dioxide including amorphous silica and quartz; silicas; metal silicates, controlled pore glasses and ceramics; metal oxides and sulfides, or combinations of these natural or synthetic and organic or inorganic materials.

The matrix backbone is preferably selected from agar-agar, agaroses, celluloses, cellulose ethers such as hydroxypropyl cellulose, carboxymethyl cellulose, polyamides such as poly(meth)acryl-amides, polyvinylalcohols, silicas, and controlled pore glasses.

Especially interesting solid phase materials as matrix backbones are e.g. agar or agarose beads such as Sepharose and Superose beads from Pharmacia Biotech, Sweden and Biogel A from Biorad, USA; dextran based beads such as Sephadex, Pharmacia Biotech; cellulose based beads and membranes such as Perloza cellulose from Secheza, Czechoslovakia; composite beads such as Sephacryl and Superdex, Pharmacia Biotech; beads of synthetic organic polymers such as Fractogel from Toso-Haas, USA; POROS media from Perceptive Biosystems, USA, Bio-Rex, Bio-Gel P and Macro Prep from Biorad, HEMA and Separon from TESSEK and Hyper D and Trisacryl media from BioSepra, USA, Enzacryl and Azlactone, 3M, USA; beads of siliceous materials such as controlled pore glass, PROSEP, from Bioprocesing, England and Spherocil, BioSepra; and coated silica composites in the form of beads or membranes such as ACTI-DISK, ACTI-MOD and CycloSep from Arbor Technologies, USA.

Typically, the solid phase matrix backbone, as well as the resulting functionalised solid phase matrix, may, e.g., be in the form of irregular particles or spherical beads, membranes or sheets, moulded surfaces, or sticks. The solid phase material may further be fully or partly permeable or completely impermeable to proteins. In a particularly interesting embodiment of the present invention, the matrix is in the form of irregular or spherical beads with sizes in the range of 1-10000 µm, preferably 10-1000 µm; such as 10-60 µm for high performance applications and such as 50-500 µm, preferably 50-300 µm, for preparative purposes. Preferred materials (polymethacrylate monoliths) for use in the present invention are disclosed in Junbauer et al. (J. Chromatogr. A 1184 (2008), 62-79).

A particular interesting form of matrix is a density controlled matrix in the form of a conglomerate comprising density controlling particles. These conglomerates are especially applicable in large scale operations for fluidised or expanded bed chromatography as well as different batch-wise chromatography techniques in non-packed columns, e.g. simple batch adsorption in stirred tanks.

Affinity ligands for the fusion protein according to the present invention may be attached to the solid phase material by any type of covalent bond known per se to be applicable for this purpose, either by a direct chemical reaction between the affinity ligand and the solid phase material or by a preceding activation of the solid phase material or of the ligand with a suitable reagent known per se making it possible to link the matrix backbone and the ligand. Examples of such suitable activating reagents are epichlorohydrin, epibromohydrin, allyl-glycidylether; bis-epoxides such as butanedioldiglycidylether; halogen-substituted aliphatic compounds such as di-chloro-propanol, divinyl sulfone; carbonyldiimidazole; aldehydes such as glutaric dialdehyde; quinones; cyanogen bromide; periodates such as sodium-meta-periodate; carbodiimides; chloro-triazines such as cyanuric chloride; sulfonyl chlorides such as tosyl chlorides and tresyl chlorides; N-hydroxy succinimides; 2-fluoro-1-methylpyridinium toluene-4-sulfonates; oxazolones; maleimides; pyridyl disulfides; and hydrazides. Among these, the activating reagents leaving a spacer group SP1 different from a single bond, e.g. epichlorohydrin, epibromohydrin, allyl-glycidylether; bis-epoxides; halogen-substituted aliphatic compounds; divinyl sulfone; aldehydes; quinones; cyanogen bromide; chloro-triazines; oxazolones; maleimides; pyridyl disulfides; and hydrazides, are preferred.

Especially interesting activating reagents are believed to be epoxy-compounds such as epichlorohydrin, allyl-glycidylether and butanedioldiglycidylether.

For peptide affinity chromatography within the scope of the present invention, any matrix useful for the immobilization of peptide ligands can be used. Preferably Fractogel epoxy (M), from Merck, Darmstadt, Germany) or equally preferred "monolithic chromatography medium" CIM-epoxy is used. The ligands can be immobilized either directly onto the chemically activated backbone of the chromatography matrix, or via a spacer or linker. In the latter case a spacer is coupled to the chromatographic matrix, said spacer is then chemically activated, in order to allow binding of the ligand. Preferably Fractogel epoxy matrices are used in combination with spacers.

In a particularly preferred embodiment of the present invention the spacer is generated by reaction of the chromatographic matrix with diaminodipropylamine (DADPA) and subsequent reaction with succinic anhydride (SA). The resulting terminal carboxy group on the spacer is chemically activated and preferably linked to a terminal amino-group. The ligand is immobilized on the matrix or on the spacer via a reactive group that it comprises. In the case of peptide ligands such reactive groups may be either the amino, carboxy or the sulfhydryl group. Within the present invention anchorage of the peptide on the matrix or the spacer via an amino bond is particularly preferred.

Preferably, the solid phase used according to the present invention is provided as affinity chromatography material, and exhibits oligopeptide ligands as disclosed in WO 2006/113958 A2.

Preferably the solid support is a chromatography column. In principle any chromatography material capable of selectively binding the first part of N^{pro} or the fusion polypeptide (comprising the second part of N^{pro} and the protein of interest) can be used within the framework of the present invention. The matrix of the chromatography may, in a preferred embodiment, be in the form of a column, however, it may also be in other forms, like beads or organic materials like polyethylene glycol modified with an affinity peptide.

Chromatography materials suitable for use within the present invention may be based on a cellulose binding domain, they may be cation exchange chromatography materials using polycationic tags like e.g. polyarginine or polylysine as well as anion exchange chromatography with polyanionic tags like e.g. polyasparagine. Accordingly within the present invention the use of an affinity chromatography material as solid support is preferably selected from the group consisting of immobilized metal ion chromatography (IMAC), cation exchange chromatography, anion exchange chromatography, cellulose binding domain chromatography and peptide affinity chromatography.

More preferably the affinity chromatography used is cation exchange chromatography, wherein the fusion polypeptide comprises a polycationic tag. Even more preferred is the use of either a polyarginine or polylysine affinity tag.

For cation exchange chromatography the expressed polypeptides preferably comprise an N-terminal polycationic tag, for example a polyarginine or polylysine tag. The solution containing the expressed polypeptide that was extracted from the host cells is (filtered) and loaded onto a column packed with any medium suitable for cation exchange chromatography such as e.g. SP Sepharose FF, CM Sepharose FF, Fractogel EMD SO3-. Preferably buffers with low conductivity are applied. After loading unbound material may be washed out and refolding may be started by introduction of a buffer with low urea concentration. At a urea concentration lower than 0.5M the target protein is cleaved off and can be eluted from the column.

Another preferred embodiment of the present invention is one, wherein the solid support is an affinity chromatography or an anion exchange chromatography and wherein the polypeptide to be bound to the column comprises a polyanionic tag. More preferably, polyasparagine is used as affinity tag.

A further preferred embodiment to achieve the desired binding properties is immobilized metal ion affinity chromatography (IMAC). Accordingly, in a preferred embodiment of the present invention the solid support is immobilized metal ion affinity chromatography (IMAC) material, and the polypeptide to be bound (especially the fusion protein comprising the second part of Npro and the protein of interest) comprises a metal chelate affinity tag. In this case the polypeptide is detected and bound by means of a metal chelate affinity tag comprised in it. In a more preferred embodiment of the present invention, the metal chelate affinity tag is a polyhistidine affinity tag. IMAC is based on the specific coordinate covalent binding between histidine or other suitable unique amino acids (either naturally present on the surface of the protein or grafted with recombinant DNA techniques) and various immobilized metal ions, such as copper, nickel, zinc, or iron. Chromatographic materials known in the art for the use in IMAC may also be useful within the present invention. In a preferred embodiment of the present invention, Ni²⁺-Chelating Sepharose Fast flow (GE Healthcare, Uppsala, SE) is used as matrix.

Alternatively, the solid support may be an immunoaffinity chromatography material, employing epitope tags as described above which are present at the N-terminus of the polypeptide and are bound to the chromatographic matrix via an antibody recognizing said tag. Another preferred affinity chromatographic material is affinity chromatography using oligopeptide ligands as disclosed in WO 2006/113958 A.

It is disclosed that the first and/or the second part comprises additional moieties, preferably an affinity tag or a refolding aid moiety, especially a His-tag, SlyD (or parts of SlyD), oligo aminoacid stretches composed of either positive or negative charged moieties, Strep-tag and/or FLAG-tag.

The present method can in principle be applied for production of any protein of interest, especially for all proteins known to be producible by the N^{pro} autoprotease technique. Since the disclosed method is suitable for large-scale manufacturing and pharmaceutical good manufacturing practice, it is preferred to produce a protein for therapeutic use in humans with the present method, preferably a human recombinant protein or a vaccination antigen. Preferred proteins of interest therefore include the proteins referred to as the 151 recombinant pharmaceuticals approved for human use by the FDA or by the EMEA by 2009 in the review of Ferrer-Miralles et al. (Microbial Cell Factories 8 (2009), 17 doi:10.1186/1475-2859-8-17), especially the products based on proteins which have already been produced in an E. coli host cell: Dukoral (Oral cholera vaccine),Pegasys (Peginterferon alfa-2a), PegIntron (Peginterferon alfa-2b), Infergen (Interferon alfacon-1), Rebetron (Interferon alfa-2b), Roferon A (Interferon alfa-2a), Viraferon (Interferon alfa-2b), ViraferonPeg (Peginterferon alfa-2b), Intron A (Interferon alfa-2b), Beromun (Tasonermin), Actimmune (Interferon gamma-1b), IPLEX (Mecasermin rinfabate recombinant), Kepivance (Palifermin), Neulasta (Pegfilgrastim), Neumega (Oprelvekin), Neupogen (Filgrastim), Humalog (Insulin lispro), Humatrope (Somatotropin), Humulin (Human insulin), Insuman (Human insulin), Lantus (Insulin glargine), Fortical (Salmon calcitpnin), Apidra (Insulin glulisine), Exubera (Human insulin), Forcaltonin (Salmon calcitonin), Forsteo (Teriparatide), Forsteo/Forteo (Teriparatide), Genotropin (Somatotropin), Glucagon, Increlex (Mecasermin), Insulin human Winthrop (Insulin human), Norditropin (Somatropin), Nutropin (Somatropin), NutropinAQ (Somatropin), Optisulin (Insulin glargine), Preotact (Human parathyroid hormone), Protropin (Somatrem), Somavert (Pegvisomant), Betaferon/Betaseron (Interferon beta-1b), Lucentis (Ranibizumab), Natrecor (Nesiritide), Rapilysin/Retavas e (Reteplase), Ontak (Denileukin diftitox), Kineret (Anakinra), and Omnitrope (Somatropin).

Preferably, step (b) is performed in a buffer, especially a phosphate, hydrogen phosphate or Tris/HCl buffer. The buffer may also comprise salts, such as NaCl, polyols, such as glycerol or carbohydrates, or detergents, such as non-ionic detergents, anionic detergents, cationic detergents, zwitterionic detergents, non-detergent sulfobetains, e.g. cetyl trimethylammonium chloride (CTAC), sodium dodecyl sulfate (SDS), lithium dodecyl sulfate (LDS) or N-lauroyl sarcosine.

The conditions for step (b) can be optimised for the individual autoprotease by applying the knowledge of the N^{pro} technology as disclosed. For example, step (b) may be performed at a pH of 5 to 11, preferably at a pH of 6 to 9.5, especially at a pH from 6.5 to 8.5. This step (b) may be performed in the presence of a buffer comprising NaCl, preferably of 50 to 1000 mM NaCl, especially of 100 to 500 mM NaCl; phosphate ions and/or hydrogen phosphate ions, preferably 5 to 500 mM phosphate and/or hydrogen phosphate, especially 10 to 200 mM phosphate and/or hydrogen phosphate; glycerol, preferably 1 to 10 % glycerol, especially 2 to 8 % glycerol; Tris/HCl, preferably 1 mM to 5 M Tris/HCl, especially 5 mM to 2 M Tris/HCl. The buffer used in step (b) can further contain one or more of the following ingredients: L-arginine, low concentrations of denaturants, polyethylene glycol, low-molecular weight non-detergent zwitterionic agents such as sulfobetaines, substituted pyridines and pyrroles, and acid substituted aminocyclohexanes.

In a preferred embodiment, basic conditions are applied, especially in step (b), e.g. by the presence of more than 5 mM NaOH or KOH, preferably more than 25 mM NaOH or KOH, more preferred of more than 50 mM NaOH or KOH, especially more than 100mM NaOH or KOH (or mixtures of KOH and NaOH); or other basic components (or mixtures thereof) resulting in a pH of more than 10, especially of a pH from 11 to 14. A specifically preferred embodiment of step (b) applies denaturing conditions to the fusion protein, preferably by a mixture of urea guanidinium hydrochloride and NaOH.

According to a preferred embodiment of the present method, the first part of the N^{pro} autoprotease and/or the second part of the N^{pro} autoprotease were generated as inclusion bodies.

The first part of the N^{pro} autoprotease and/or the second part of the N^{pro} autoprotease can be renatured before step (b); according to a preferred embodiment, however, the renaturing of the first part of the N^{pro} autoprotease and/or the second part of the N^{pro} autoprotease takes place simultaneously in step (b).

The first part of the N^{pro} autoprotease and/or the second part of the N^{pro} autoprotease may also be purified before recovering step (c); accordingly, it is preferred to purify or at least partially purify the first part of the N^{pro} autoprotease and/or the second part of the N^{pro} autoprotease before step (b).

Such purification may be performed by any suitable purification method available. It is, however, preferred that the purification is performed by affinity purification, preferably affinity chromatography or affinity precipitation.

Preferably, the first part of the N^{pro} autoprotease is provided in step (a) in immobilised form, preferably by covalent binding to a solid support, especially via the N-terminus.

It is possible to produce the first part of the N^{pro} autoprotease and the second part of the N^{pro} autoprotease in different cells (cell lines), i.e. separately. It is also possible that the first part of the N^{pro} autoprotease and the second part of the N^{pro} autoprotease were produced in the same cells so that they can be expressed and obtained simultaneously. In such disclosures, it is often preferred that expression takes place under condition where (autoproteolytic) cleavage (by complementation) is prevented by applying conditions that prevent complementation and/or autoproteolytic activity of N^{pro} (if complemented). On the other hand, it is also possible to allow complementation and autoproteolysis to perform within the production cell (so that step (b) is performed within the producing cell).

The two parts of Npro as defined herein may be provided by established techniques, e.g. by way of inclusion bodies (especially the second part with the protein of interest). Inclusion bodies can be solubilised and refolded by such established techniques e.g. as disclosed in WO 01/11057 A, WO 01/11056 A, WO 2006/113957 A, WO 2006/113958 A, WO 2006/113959 A, and Achmüller et al., Nat. Meth. 4 (2007), 1037-1043. The solubilised/refolded molecules can then be applied to the complementing step. It is also possible to combine the solubilisation/refolding with the complementation step, preferably by dilution in the presence of the first part of the autoprotease or by solubilisation/refolding on a solid phase in the presence of the first part of the autoprotease.

According to another aspect, the present invention relates to an N^{pro} autoprotease fragment consisting of amino acid 22 to 30 of Npro autoprotease, preferably amino acid 22 to 75, more preferred amino acid 22 to 112, 1 to 75 or 1 to 30, especially amino acid 1 to 112.

Another aspect of the present invention relates to an N^{pro} autoprotease fragment part being fused by a peptidic bond to a protein of interest but not exhibiting a proteolytic activity, consisting of amino acid 31 to 168 of Npro autoprotease, preferably amino acid 76 to 168, especially amino acid 113 to 168.

According to another aspect, the present invention relates to an Npro autoprotease fragment consisting of amino acid 1 to 112 of Npro autoprotease, preferably amino acid 22 to 112, 1 to 75 or 1 to 30, especially amino acid 22 to 30, 22 to 75.

Another aspect of the present invention relates to an Npro autoprotease fragment part being fused by a peptidic bond to a protein of interest but not exhibiting a proteolytic activity, consisting of amino acid 31 to 168 of Npro autoprotease, preferably amino acid 76 to 168, especially amino acid 113 to 168. Even more preferred Npro fragments fused to a protein of interest contain, as N-terminal sequence, amino acids 105 (up to 160) to 168 of an N^{pro} autoprotease sequence, i.e. fusion proteins of a protein of interest containing, as N-terminal sequence amino acids 105-168, 106-168, 107-168, 108-168, 109-168, 110-168, 111-168, 112-168, 113-168, 114-168, 115-168, 116-168, 117-168, 118-168, 119-168, 120-168, 121-168, 122-168, 123-168, 124-168, 125-168, 126-168, 127-168, 128-168, 129-168, 130-168, 131-168, 132-168, 133-168, 134-168, 135-168, 136-168, 137-168, 138-168, 139-168, 140-168, 141-168, 142-168, 143-168, 144-168, 145-168, 146-168, 147-168, 148-168, 149-168, 150-168, 151-168, 152-168, 153-168, 154-168, 155-168, 156-168, 157-168, 158-168 159-168, or 160-168 of an N^{pro} autoprotease sequence. As already stated above, the preferred cleavage sites dividing the first part of the N^{pro} autoprotease from the second part of the N^{pro} autoprotease are from amino acid residue 30 to 160, more preferred from amino acid residue 65 to 85, 105 to 120, 140 to 160, especially from 107 to 115 and from 140 to 154, of an N^{pro} autoprotease sequence.

With such fusion polypeptides comprising a C-terminal fragment of Npro ("C-Npro") and a protein of interest, it is also possible to raise titers in recombinant production of the protein of interest. Moreover, such C-Npro can be used (with or without affinity tags) for affinity purification as disclosed herein (and even without complementation). According to another aspect, the present invention therefore also relates to a method wherein a fusion polypeptide comprising a C-terminal fragment of Npro ("C-Npro") and a protein of interest is recombinantly expressed (e.g. in inclusion bodies, preferably in E. coli), the fusion polypeptide is purified, especially via affinity purification of the C-Npro moiety or, if present, an affinity tag (or also via the protein of interest moiety), and wherein the C-Npro moiety is cleaved off by a protease (activity), preferably either by complementation with the N-terminal part of Npro as described above or by the activity of an external protease being specific for the peptidic bond between the C-Npro and the protein of interest.

The present invention also relates to an expression vector encoding for an N^{pro} autoprotease fragment (first part) or fusion protein (second part) according to the present invention. In the expression vector to be employed in the process according to the present invention, the fragment or fusion polypeptide is operably linked to at least one expression control sequence. Expression control sequences are, in particular, promoters (such as the lac, tac, T3, T7, trp, gac, vhb, lambda pL or phoA promoter), ribosome binding sites (for example natural ribosome binding sites which belong to the abovementioned promoters, cro or synthetic ribosome binding sites), or transcription terminators (for example rrnB T1T2 or bla).

The vector may also contain sequences encoding fusion domains, as described below, that are present at the N-terminal end of the fusion polypeptide and that are required for its binding to the affinity chromatography system, e.g. polyamino acids like polylysine or, for immunoaffinity chromatogography, so-called "epitope tags", which are usually short peptide sequences for which a specific antibody is available. Well known epitope tags for which specific monoclonal antibodies are readily available include FLAG, influenza virus haemagglutinin (HA), and c-myc tags.

In a preferred embodiment of the present invention, the expression vector is a plasmid.

Another aspect of the present invention relates to a host cell, preferably a prokaryotic host cell, especially an E. coli host cell, containing an expression vector according to the present invention. The transformed bacterial host cell, i.e. the expression strain, is cultivated in accordance with microbiological practice known per se. The host strain is generally brought up starting from a single colony on a nutrient medium, but it is also possible to employ cryo-preserved cell suspensions (cell banks). The strain is generally cultivated in a multistage process in order to obtain sufficient biomass for further use.

On a small scale, this can take place in shaken flasks, it being possible in most cases to employ a complex medium (for example LB broth). However, it is also possible to use defined media (for example citrate medium). Since in the preferred embodiment of the present invention it is intended that especially the expressed fusion polypeptide comprising the second part of N^{pro} is in the form of insoluble inclusion bodies, the culture will in these cases be carried out at relatively high temperature (for example 30°C or 37°C). Inducible systems are particularly suitable for producing inclusion bodies (for example with the trp, lac, tac or phoA promoter).

On a larger scale, the multistage system consists of a plurality of bioreactors (fermenters), it being preferred to employ defined nutrient media. In addition, it is possible greatly to increase biomass and product formation by metering in particular nutrients (fed batch). Otherwise, the process is analogous to the shaken flask. In the process according to the present invention, the inclusion bodies are isolated from the host cell in a manner known per se. For example, after the fermentation has taken place, the host cells are harvested by centrifugation, micro filtration, flocculation or a combination thereof, preferably by centrifugation. The wet cell mass is disintegrated by mechanical, chemical or physical means such as high pressure homogenizer, beads mills, French press, Hughes press, osmotic shock, detergents, enzymatic lysis or a combination thereof. Preferably, disruption of the cells takes place by high pressure homogenization. In the preferred embodiment where the recombinant fusion polypeptide is deposited as inclusion bodies, the inclusion bodies can be obtained for example by means of high-pressure dispersion or, preferably, by a simple centrifugation at low rotor speed. The inclusion bodies are separated by centrifugation or microfiltration or a combination thereof. The purity in relation to the desired polypeptide of interest can then be improved by multiple resuspension of the inclusion bodies in various buffers, for example in the presence of NaCl (for example 0.5 1.0 M) and/or detergent (for example Triton X 100). Preferably the purity of the inclusion body preparation is improved by several washing steps with various buffers (e.g. 0.5 % Deoxycholate followed by two times 1 M NaCl solution and finally distilled water). This usually results in removal of most of the foreign polypeptides from the inclusion bodies.

According to another aspect, the present invention relates to a solid support comprising a first part (i.e. an N-terminal part) of an N^{pro} autoprotease, wherein complementation of said first part with the C-terminal part (second part) of the N^{pro} autoprotease forms an autoproteolytically active N^{pro} autoprotease. This solid support is defined as mentioned above and comprises an N-terminal part of the N^{pro} autoprotease bound thereto. This N-terminal part of the N^{pro} autoprotease bound to the solid support can then be subjected to complementation according to the present invention. The binding of the N-terminal part of the N^{pro} autoprotease to the solid support is preferably effected by adsorption or covalent binding.

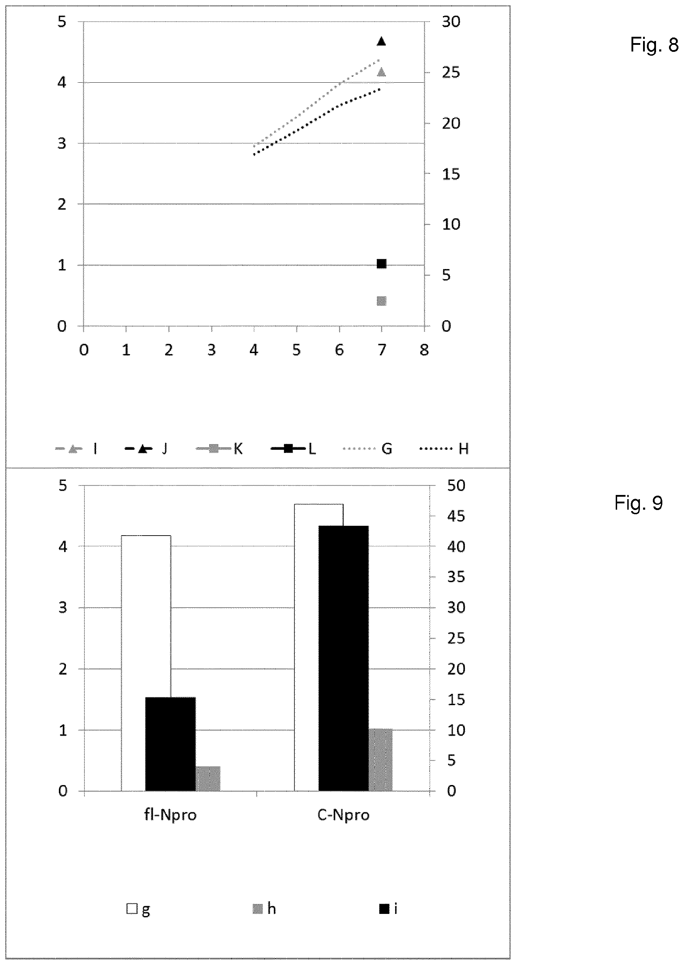

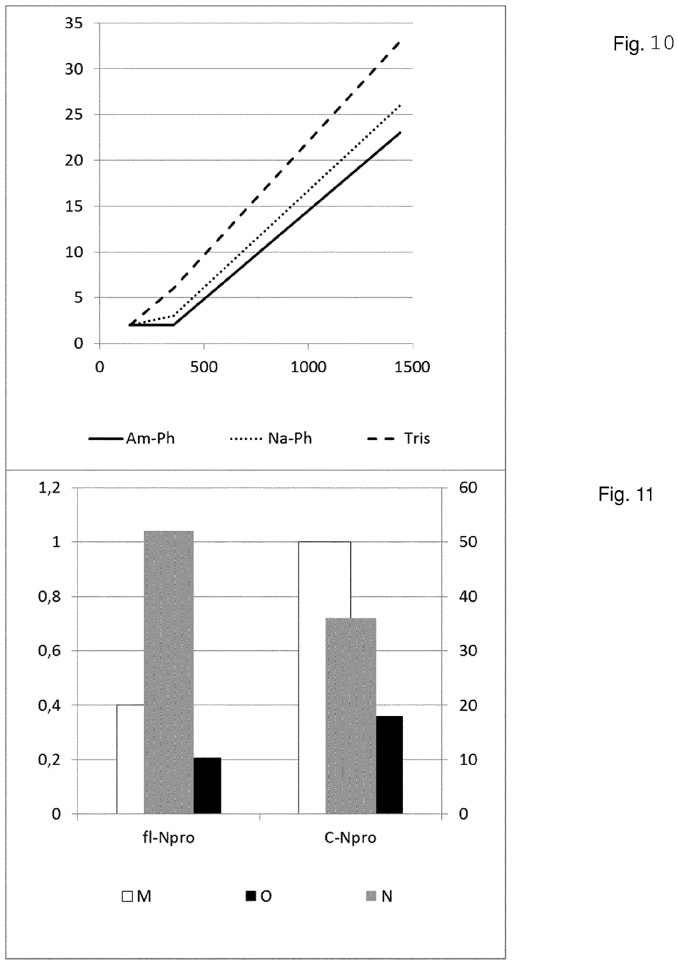

Alternatively, disclosed is a solid support comprising a second part (i.e. a C-terminal part) of an N^{pro} autoprotease, said second part being fused by a peptidic bond to a protein of interest but not exhibiting a proteolytic activity (but being complementable with a first part of an N^{pro} autoprotease according to the present invention). This solid support is defined as mentioned above and comprises a C-terminal part of the N^{pro} autoprotease being fused by a peptidic bond to a protein of interest but not exhibiting a proteolytic activity bound thereto. This C-terminal part of the N^{pro} autoprotease bound to the solid support can then be subjected to complementation according to the present invention. Again, the binding of the C-terminal part of the N^{pro} autoprotease to the solid support is preferably effected by adsorption or covalent binding.

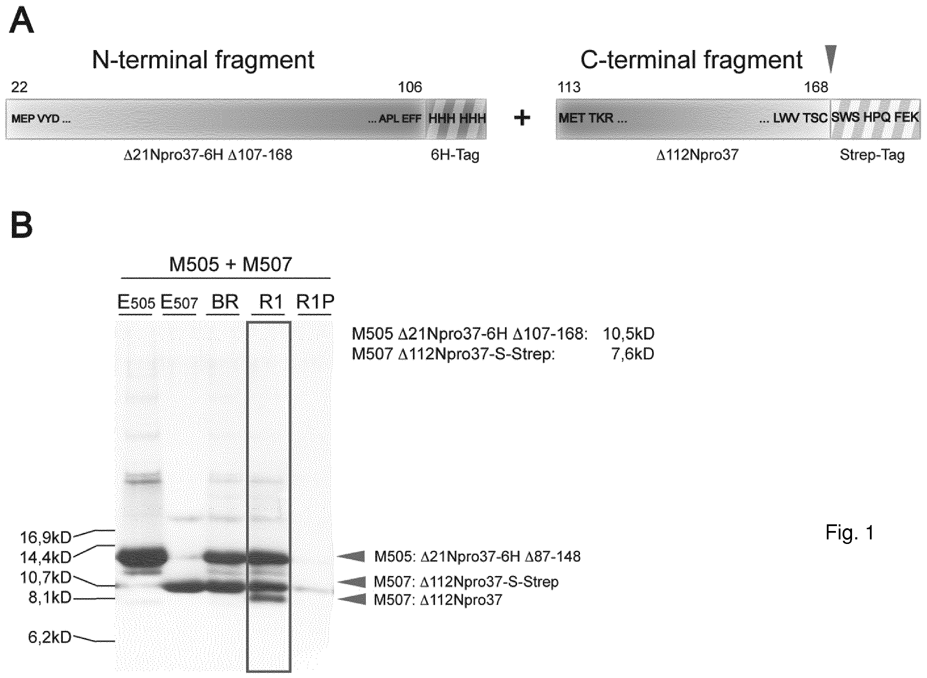

Figure 1: Complementation at position 112 with Npro fragments. A: scheme of N^{pro} fragments D21N^{pro}37-6H D107-168 (N^{pro}505) and D112N^{pro}37-S-Strep (N^{pro}507). B: Tris-tricine SDS-PAGE of the complementation at position 112. E505...eluted fraction of N^{pro}505; E507...eluted fraction of N^{pro}507; BR...before refolding; R...Refolding; RP...refolding pellet; Amount of protein in PAGE samples: E505: 23µg, E507: 17µg, BR: 35µg, R: 34µg.

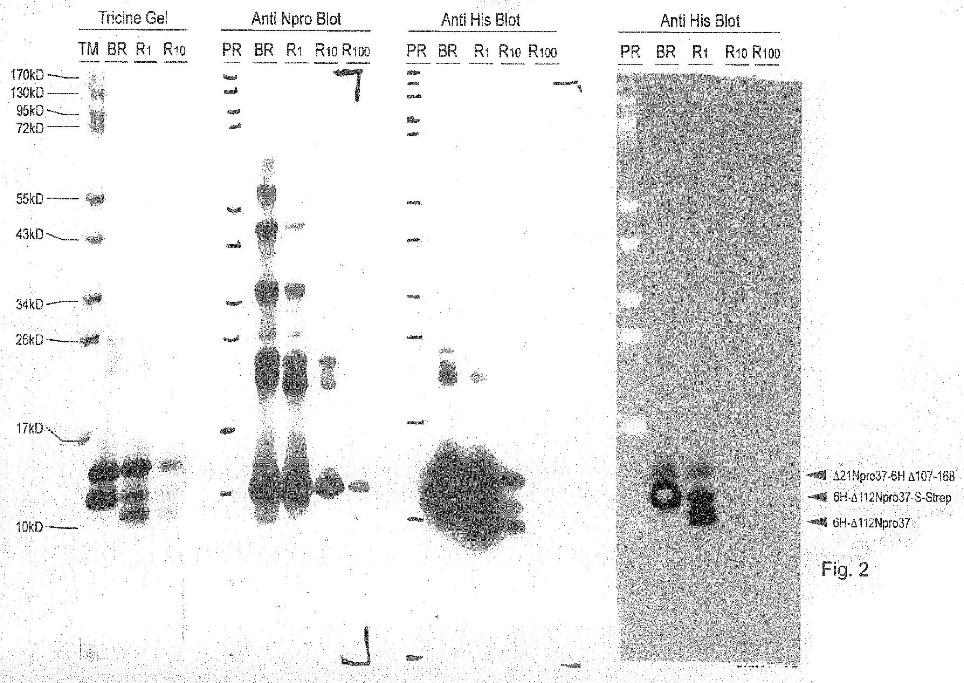

Figure 2: Western blot of the complementation of N^{pro}505 D21N^{pro}37-6H D107-168 (10,5kD) with N^{pro}506 D92N^{pro}37-S-Strep (8, 3kD). BR...before refolding; R1...Refolding; R10...1 : 10 dilution of the refolding sample; R100...1:100 dilution of the refolding sample; Amount of protein in PAGE samples: BR: 62µg, R1: 62µg, R10: 6µg. Anti Npro Blot: anti-NproEDDIE (animal 10201) antibody, produced in goat, spec. IgG from 50ml antiserum (BioGenes GmbH) Anti His Blot: HisProbe HRP (Pierce). The left Anti His Blot was classically developed onto an X-ray film while for the right Anti His Blot an imager from Proxima was used.

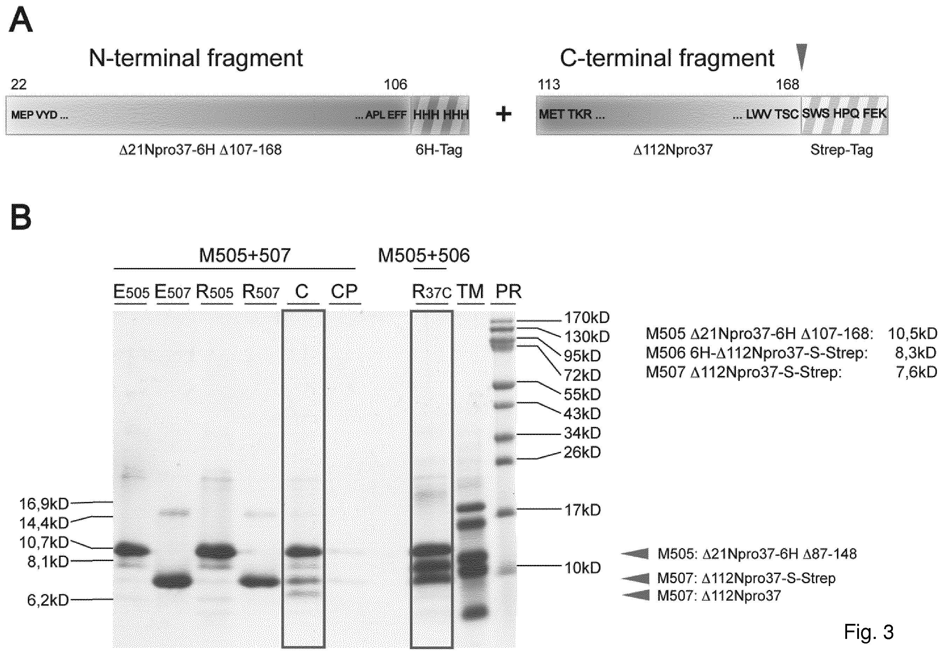

Figure 3: Complementation at position 112 with renaturated N^{pro} fragments. A: scheme of N^{pro} fragments D21N^{pro}37-6H D107-168 (N^{pro}505) and D112N^{pro}37-S-Strep (N^{pro}507). B: Tris-tricine SDS-PAGE of the complementation at position 112 with renaturated N^{pro} fragments. E₅₀₅ ... eluted fraction of N^{pro}505; E₅₀₇ ... eluted fraction of N^{pro}507; BR...before refolding; R...Refolding; RP...refolding pellet; R_{37c}...refolding at 37°C. Amount of protein in PAGE samples: E₅₀₅ : 22µg, E₅₀₇: 14µg, R₅₀₅: 22µg, R₅₀₇: 14µg, R: 18µg.

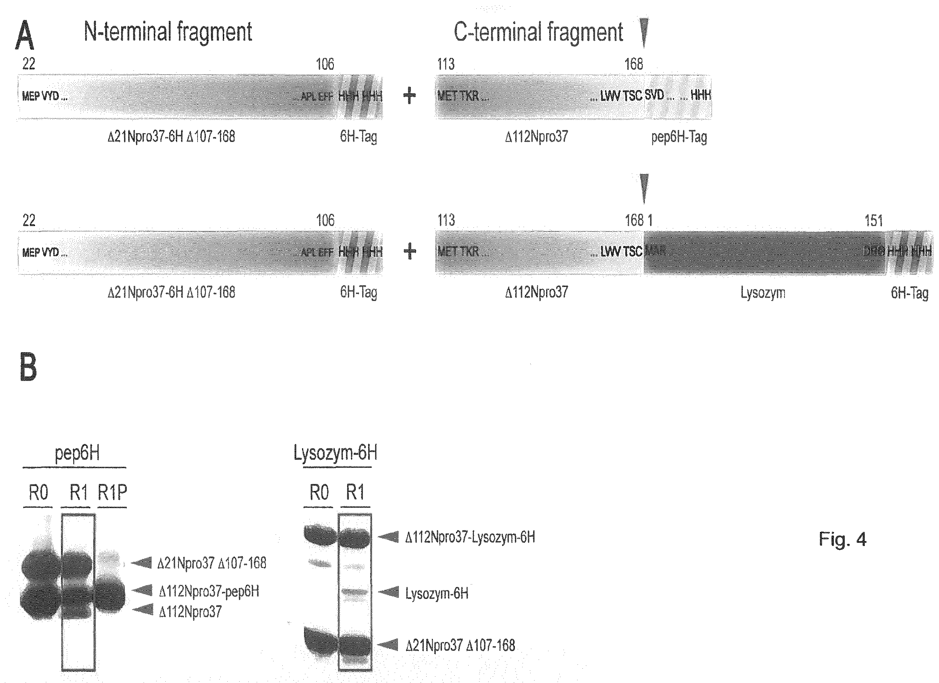

Figure 4: Complementation at amino acid position 112 with D112N^{pro}37-pep6H and D112N^{pro}37-Lysozyme-6H. A: scheme of D112N^{pro}37-pep6H and D112N^{pro}37-Lysozyme-6H. B: SDS-PAGE gels of the refoldings in different buffers.
   R0...N^{pro} before refolding; R1...refolding after 1 day; R1P...Pellet of refolding after 1 day.

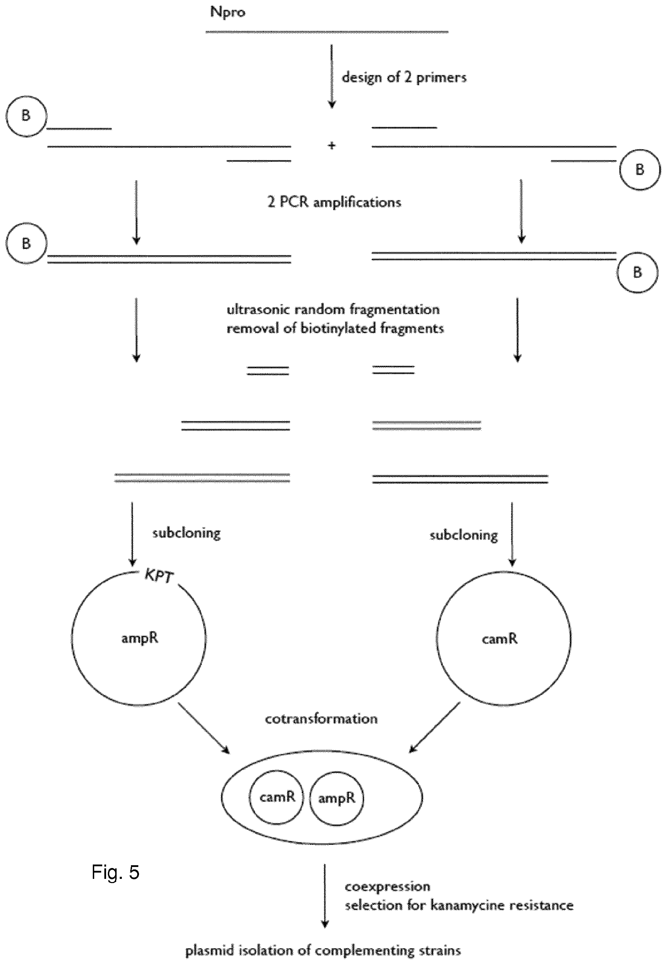

Figure 5: General random approach for selecting all possible complementing protein fragment combinations of N^{pro}.

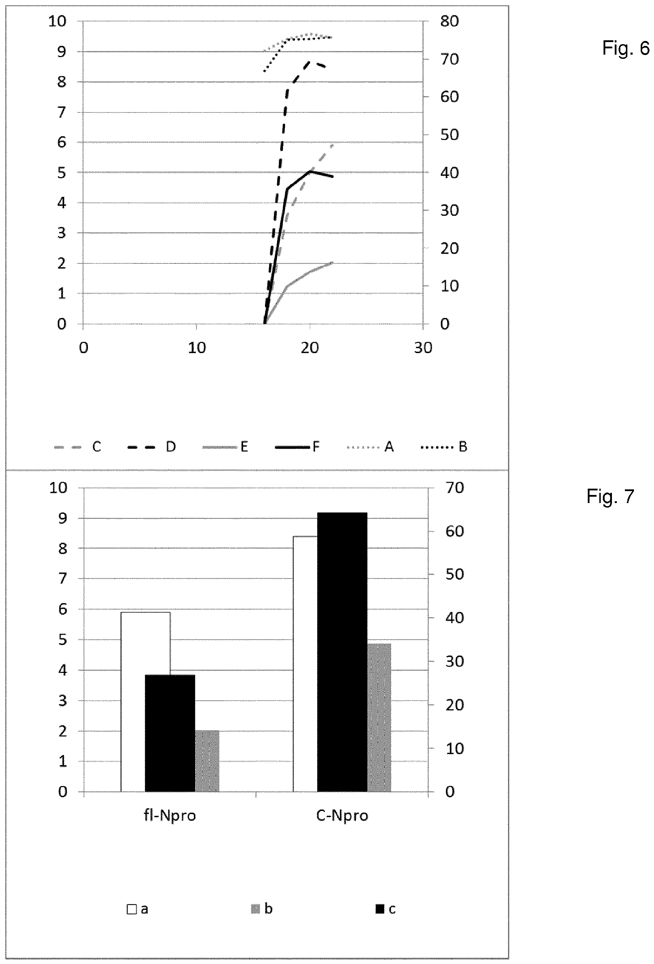

Figure 6: Fermentation of D21N^{pro}37-MCP-1 (SEQ.ID.NO. 17) and D112N^{pro}37-MCP-1 (SEQ. ID. NO. 16): Course of formation of biomass (DCW), titer of the full length N^{pro} fusion with MCP-1 and titer of the C-terminal N^{pro} fusion with MCP-1 during fermentation and the titers for MCP-1 within the fusion proteins x-axis stands for the feed time in [h]
   left y-axis stands for the titer in [g/L]
   right y-axis stands for the DCW (dry cell weight) in [g/L]
   A: DCW of cells producing the full length N^{pro} fusion protein, D21N^{pro}37-MCP-1 (SEQ.ID.NO. 17)
   B: DCW of cells producing the C-terminal N^{pro} fusion protein, D112N^{pro}37-MCP-1 (SEQ. ID. NO. 16)
   C: titer of full length N^{pro} fusion protein, D21N^{pro}37-MCP-1 (SEQ.ID.NO. 17)
   D: titer of the C-terminal N^{pro} fusion protein, D112N^{pro}37-MCP-1 (SEQ. ID. NO. 16)
   E: titer of MCP-1 within the full length N^{pro} fusion protein, D21N^{pro}37-MCP-1 (SEQ.ID.NO. 17)
   F: titer of MCP-1 within the C-terminal N^{pro} fusion protein, D112N^{pro}37-MCP-1 (SEQ. ID. NO. 16)
Figure 7: Fermentation of D21N^{pro}37-MCP-1 (SEQ.ID.NO. 17) and D112N^{pro}37-MCP-1 (SEQ. ID. NO. 16) : Comparison of the titer of the fusion proteins, the titer of MCP-1 and the specific titer of MCP-1 for the full length N^{pro} fusion and the C-terminal N^{pro} fusion after fermentation.
   Left y-axis stands for the titer in [g/L]
   Right y-axis stands for the specific titer in [mg /g DCW] fl-Npro stands for the full length N^{pro} fusion protein, D21N^{pro}37-MCP-1 (SEQ.ID.NO. 17)
   C-Npro stands for the C-terminal N^{pro} fusion protein, D112N^{pro}37-MCP-1 (SEQ. ID. NO. 16)
   a: titers of the fusion proteins
   b: titers of MCP-1 within the fusion proteins
   c: specific titer of MCP-1
Figure 8: Fermentation of D21N^{pro}37-pep6His (SEQ.ID.NO. 19) and D112N^{pro}37-pep6His (SEQ. ID. NO. 4) : Course of formation of biomass (DCW), titer of the full length N^{pro} fusion with pep6His and titer of the C-terminal N^{pro} fusion with pep6His during fermentation and the titers for pep6His within the fusion proteins.
   x-axis stands for the feed time in [h]
   left y-axis stands for the titer in [g/L]
   right y-axis stands for the DCW (dry cell weight) in [g/L]
   G: DCW of cells producing the full length N^{pro} fusion protein, D21N^{pro}37-pep6His (SEQ.ID.NO. 19)
   H: DCW of cells producing the C-terminal N^{pro} fusion protein, D112N^{pro}37-pep6His (SEQ. ID. NO. 4)
   I: titer of full length N^{pro} fusion protein, D21N^{pro}37-pep6His (SEQ.ID.NO. 19)
   J: titer of the C-terminal N^{pro} fusion protein, D112N^{pro}37-pep6His (SEQ. ID. NO. 4)
   K: titer of MCP-1 within the full length N^{pro} fusion protein, D21N^{pro}37-pep6His (SEQ.ID.NO. 19)
   L: titer of MCP-1 within the C-terminal N^{pro} fusion protein, D112N^{pro}37-pep6His (SEQ. ID. NO. 4)
Figure 9: Fermentation of D21N^{pro}37-pep6His (SEQ.ID.NO. 19) and D112N^{pro}37-pep6His (SEQ. ID. NO. 4): Comparison of the titer of the fusion proteins, the titer of pep6His and the scecific titer of pep6His for the full length N^{pro} fusion and the C-terminal N^{pro} fusion after fermentation.
   Left y-axis stands for the titer in [g/L]
   Right y-axis stands for the specific titer in [mg /g DCW]
   fl-Npro stands for the full length N^{pro} fusion protein, D21N^{pro}37-pep6His (SEQ.ID.NO. 19)
   C-Npro stands for the C-terminal N^{pro} fusion protein, D112N^{pro}37-pep6His (SEQ. ID. NO. 4)
   g: titers of the fusion proteins
   h: titers of pep6His within the fusion proteins
   i: specific titer of pep6His
Figure 10: Cleavage of the complementation system (D21N^{pro}37-6H D107-168, SEQ.ID.NO. 1 and D112-N^{pro}37-pep6His, SEQ.ID. NO. 4) X-axis stands for the time in [min]
   Y-axis stands for the cleavage yield in [%]
   Am-Ph: cleavage yield for the complemmentation system in Ammonium phosphate buffer as described in table 3
   Na-Ph: cleavage yield for the complemmentation system in Sodium phosphate buffer as described in table 3
   Tris: cleavage of for the complemmentation system in TRIS buffer as described in table 3
Figure 11: Comparison of the titer of pep6His, the cleavage yield of the fusion proteins and the overall productivity for the full length N^{pro} fusion protein, D21N^{pro}37-pep6His (SEQ.ID.NO. D) and the C-terminal N^{pro} fusion protein, D112N^{pro}37-pep6His (SEQ. ID. NO. 4)
   Left y-axis stands for the titer in [g/L] and the overall productivity after cleavage (as explained in Example 8) in [g/L] Right y-axis stands for the cleavage yield in [%]
   fl-Npro stands for the full length N^{pro} fusion protein, D21N^{pro}37-pep6His (SEQ.ID.NO. 19)
   C-Npro stands for the C-terminal N^{pro} fusion protein, D112N^{pro}37-pep6His (SEQ. ID. NO. 4)
   M: titers of pep6His within the fusion proteins
   N: cleavage yield
   O: overal productivity for pep6His after cleavage [g pep6His after cleavage per L culture broth]

### Examples

### Materials and methods

### Preparation of fragments

DNA fragment coding for amino acid 22 to 106 of D21N^{pro}37 was amplified from a pET30a vector harboring D21N^{pro}37-pep6H with the forward primer 5'-GAAATTAATACGACTCACTATAGG-3' (SEQ.ID.NO.6), which has an NdeI restriction site and a reverse primer 5'-ATATAGTCGACTTATTAAAAGAATTCCAGAGG-3' (SEQ.ID.NO.7), which has a SalI restriction site as well as two TAA stop codons. Addition of a C-terminal 6H tag was achieved using the reverse primer 5'-ATAGTCGACTTATTAGTGATGGTGATGGTGATGAAAGAATTCCAGAGG-3' (SEQ.ID.NO.8). C-terminal fragments ranging from amino acid 113 to 168 in combination with a C-terminal Strep tag were generated without N-terminal 6H tag using forward primer 5'-TATACATATGGAAACCACTAAACGTATTGGC-3' (SEQ.ID.NO.9) and with 6H tag using forward primer 5'-TATACATATGCATCACCATCACCATCACGAAACCACTAAACGTATTGGC-3' (SEQ.ID.NO.10) and reverse primer 5'-GTTATGCTAGTTATTGCTCAGCGG-3' (SEQ.ID.NO.11). D21N^{pro}37-pep6H in a pET30a vector serves as template. The PCR products obtained were digested with NdeI and SalI, and ligated into a pET30a vector restricted with the same enzymes.

For expression of N^{pro} fragments *E. coli* strain BL21 (DE3) was used. *E. coli* BL21 (DE3) was transformed by electroporation and grown o/n at 37°C. After a 1:100 dilution, *E. coli* was grown until an OD₆₀₀ of about 0.5 was reached. Subsequently protein expression was induced by addition of 1mM IPTG to the suspension. After an incubation for 4 hours at 37°C, 225rpm the cells were harvested by centrifugation and lysis was achieved by French press treatment. IBs were collected by centrifugation.

N-terminal and C-terminal N^{pro} fragments were expressed separately. After solubilisation of IBs in a NaH₂PO₄ buffer containing 5M GdnHCl, 100mM NaCl, 10mM MTG, and 50mM NaH₂PO₄ buffer (pH 7.0) N^{pro} fragments were purified either by a Ni-NTA column or by a Strep-Tactin column depending on the fusion tag.

### Complementation during renaturation

About 1mg of the eluted N-terminal fragment D21N^{pro}37-6H D107-168 and 1mg of the C-terminal fragment D112N^{pro}37-S-Strep were pooled and refolded by 1:25 dilution in a NaH₂PO₄ buffer (pH 7.0) containing 30% glycerol, 100mM NaCl, 10mM MTG. After one day incubation at 20°C precipitated protein was pelleted by centrifugation at 5000g for 1 hour. A sample of the soluble fraction as well as from the pellet was loaded onto a denaturating Tris-Tricine PAGE gel (fig. 1).

### Complementation of renatured fragments

To make sure that the cleavage of N^{pro} is by complementation of two fragments and not by an intrinsic catalytic activity of the C-terminal fragment, another experiment was carried out. Therefore, the N-terminal fragment D21N^{pro}37-6H D107-168 and the C-terminal fragment D112N^{pro}37-S-Strep were expressed separately. After solubilisation of IBs, D21N^{pro}37-6H D107-168 was purified by a Ni-NTA column while D112N^{pro}37-S-Strep was purified by a Strep-Tactin resin. Eluted fractions containing N^{pro} were pooled. Then about 1mg of D21N^{pro}37-6H D107-168 and about 1mg of D112N^{pro}37-S-Strep were refolded by 1:25 dilution separately. After incubation at 20°C o/n both refoldings were combined and incubated a further day at 20°C. Afterwards precipitated protein was pelleted by centrifugation at 5000g for 1 hour. A sample of the soluble fraction as well as from the pellet was loaded onto a denaturating Tris-Tricine PAGE gel (fig. 2).

### Example 1: Complementation during renaturation

A denaturating Tris-Tricine PAGE of the complementation of denaturated N-terminal fragment D21N^{pro}37-6H D107-168 in combination with denaturated C-terminal fragment D112N^{pro}37-S-Strep shows that both parts complement each other and form a functional protease (fig. 1).

Western blots were conducted as a confirmation of the complementation experiments (fig. 2). It looks like the polyclonal anti N^{pro} antibody recognizes just the N-terminal fragment of N^{pro} while there is no signal from the C-terminal fragment. However, all three N^{pro} fragments (D21N^{pro}37-6H D107-168, 6H-D92N^{pro}37-S-Strep and 6H-D92N^{pro}37) could be identified by an India His blot. This verifies the operating principle of complementing two N^{pro} fragments.

### Example 2: Complementation of renatured fragments

A denaturating Tris-Tricine PAGE of the complementation of renatured N-terminal fragment D21N^{pro}37-6H D107-168 in combination with renatured C-terminal fragment D112N^{pro}37-S-Strep shows that the C-terminal fragment alone is not able to cleave off the Strep tag. Only by combining both parts of N^{pro} an active autoprotease is formed (fig. 3).

A further complementation was carried out at 37°C with fragments D21N^{pro}37-6H D107-168 and 6H-D112N^{pro}37-S-Strep. Both fragments were united in denaturating buffer and renaturated together by rapid 1:25 dilution (fig. 3). As the PAGE gel shows, the complementation works at 37°C as well.

### Example 3: Complementation with different C-terminal fusion partners

Further complementation experiments were carried out with different fusion partners coupled to the C-terminal fragment. A cleavage of the autoprotease was observed with D112N^{pro}37 in combination with pep6H and Lysozym-6H (fig. 4). Although the cleavage rate in combination with Lysozym-6H is very low, it shows that the principle of complementing two parts of N^{pro} works with other fusion partners than Strep.

### Example 4: General random approach to select for all possible complementing protein fragment combinations of Npro

In this example, a method for the general determination of possible complementing protein fragment combination of N^{pro}-Protein of interest-combination is disclosed.

This approach is based on the complementation of random N-terminal fragments of N^{pro} with random C-terminal fragments in fusion with kanamycinephosphotransferase (KPT). It is published (PH.D. Thesis Philipp Wechner, 2005, Leopold-Franzens Universität Innsbruck) that protein-fusions to the N-terminus of KPT inhibit its enzymatic activity and render bacteria expressing such a fusion sensitive to kanamycine - thus, the selection strategy would rely on the fact that only on reconstitution of active Npro from the random fragments by complementation, cleavage could occur and respective bacteria expressing active KPT could be selected with kanamycine.

This approach requires only 2 PCR primers (with integrated suitable restriction sites), each with and without biotin label, to generate 2 full-lenght N^{pro} coding fragments that are labeled with biotin either at the N or C-terminus (Fig. 5). Separate fragmentation of these N^{pro} coding sequences by ultrasonic treatment , repair/polishing of the ends by Pfu-Polymerase and removal of the Biotin-labeled species from the two reactions by passage through a streptavidine column results in two series of N^{pro}-fragments with either N- or C-terminal deletions and with blunt ends at the N- or C-terminus, respectively. After appropriate cutting of these fragment-pools within the original primers and subcloning the two series in two expression vectors that have different selection markers (not KTP) generates two N^{pro}, fragment libraries that can be used to identify fragment-combinations that can activate kanamycine-resistance by triggering the cleavage of the C-terminal N^{pro} fragment from the KPT moeity, simply by cotransfecting the two vector libraries with the fragment series into a kanamycine sensitive host and selecting for complementation with kanamycine. Complementing N^{pro} fragment combinations can then be simply analysed by isolation of the plasmid combination from the resistant strain and direct sequencing of the plasmid mixture with respective specific primers. This is a robust straightforward method to identify all possible complementing pairs of N^{pro}-fragments.

### Materials:

### Primers:

Npro Forward NdeI: CGC GAC ATA TGG AAC TCA ATC ATT TCG AAC (+/-Biotin at the 5'nucleotide)
Npro 150 Reverse SpeI GCT GCA ACT AGT GAC C (+/- Biotin at the 5'nucleotide)

### Vectors:

For the cloning of the C-terminal fragments as N-terminal fusions with KTP:
pET-30a (Novagen) with NdeI-N^{pro}-SpeI-KTP-SalI cut with AsuII. Filled up with T4 polymerase then cut with SpeI to integrate the C-terminal fragments which are 5'blunt-ended and 3' cut with SpeI.

For cloning of the N-terminal fragments:
pCM470_dsbC cut with HindIII, filled up with T4 polymerase and then cut with NdeI to provide a ATG followed by a blunt end for the integration of the 3'blunt-ended N-terminal fragments to be cloned in front of the stop codon TGA following the HindIII site.

### Example 5: Expression of D21N^{pro}37-MCP-1, SEQ. ID. NO. 17 (full length N^{pro} fusion) and the complementation fragment, D112N^{pro}37-MCP-1, SEQ. ID.NO. 16 (C-terminal N^{pro}-fusion), with E. coli in fed batch mode

### Generation of Bacterial Strains and Description of Recombinant Proteins

### Molecular weights:

D112N ^{pro}37-MCP-1 (SEQ. ID. NO. 16) : 15 kD
D21N^{pro}37-MCP-1 (SEQ.ID.NO. 17): 25.3 kD
MCP-1(SEQ. ID. NO. 18): 8.7 kD

### Sequences:

D112Npro37-MCP-1 (SEQ: ID. NO. 16) 
METTKRIGRVTGSDGKLYHIYVEVDGEILLKLAKRGTPRTLKWTRNTTNCPLWVTSCQPDAINA
PVTCCYNFTNRKISVQRLASYRRITSSKCPKEAVIFKTIVAKEICADPKQKWVQDSMDHLDKQT
QTPKT
D21Npro37-MCP-1 (SEQ. ID. NO. 17) 
MEPVYDTAGRPLFGNPSEVHPQSTLKLPHDRGRGDIRTTLRDLPRKGDCRSGNHLGPVSGIYIK
PGPVYYQDYTGPVYHRAPLEFFDETQFEETTKRIGRVTGSDGKLYHIYVEVDGEILLKLAKRGT
PRTLKWTRNTTNCPLWVTSCQPDAINAPVTCCYNFTNRKISVQRLASYRRITSSKCPKEAVIFK
TIVAKEICADPKQKWVQDSMDHLDKQTQTPKT
MCP-1(SEQ. ID. NO 18)

QPDAINAPVTCCYNFTNRKISVQRLASYRRITSSKCPKEAVIFKTIVAKEICADPKQKWVQDSM
DHLDKQTQTPKT

**Table 1: List of generated expression strains**

| Protein | Strain | Plasmid | Promoter |
|---|---|---|---|
| D112Npro37-MCP-1 (SEQ. ID. NO. 16) | Bl21 (DE3) Novagen # 6950 | pET30a(+) from Novagen | T7 |
| D21Npro37-MCP-1 (SEQ. ID. NO. 17) | Bl21 (DE3) Novagen # 6950 | pET30a(+) from Novagen | T7 |

Codon optimized genes of interest (GOIs) were synthesized (Geneart AG, Germany) and provided in the Geneart plasmids with an NdeI restriction site and an EcoRI restriction site as well as 2 TAA stop codons. The GOI DNAs obtained were digested with NdeI and EcoRI, and ligated into a pET30a vector (Novagen, Merck Millipore)restricted with the same enzymes.

For expression of Npro fragments E. coli strain BL21 (DE3) was used. E. coli BL21 (DE3) was transformed and grown o/n at 37°C.

3 clones were picked, resuspended in Luria-Bertani (LB) medium (Bertani, et.al 1951), grown at 37°C until an optical density (OD550nm)of 0.2 - 0.5 and frozen as glycerol stock, all separately.

For expression evaluation clone screenings were performed: LB medium was inoculated from glycerol stock and grown overnight at 37°C. For the clone screening production culture LB medium was inoculated with a seed culture to an OD550nm of 0.2, grown until OD 1. Subsequently protein expression was induced by addition of 1mM IPTG (Isopropyl β-D-1-thiogalactopyranoside) to the suspension. After incubation for 4 hours at 37°C, the cell pellets were harvested by centrifugation, stored at -20°C and analyzed for productivity by SDS (Sodium Dodecyl Sulfate) Page (Polyacrylamine gel electrophoresis) as follows:
- Sample preparation: BugBuster Extraction Reagent (Novagen-Merck) plus Lysonase Bioprocessing Reagent mix (Novagen-Merck)
- Gel: NuPage 12% Bis Tris, 1.0mm (Invitrogen)
- Sample buffer: NuPAGE LDS Sample Buffer (Invitrogen)
- Reducing agent: 2-Mercaptoethanol (Sigma-Aldrich)
- Running buffer: NuPAGE MES SDS Running Buffer (Invitrogen)
- Detection: SimplyBlue SafeStain (Invitrogen)

### Cultivation Mode and Process Analysis

### Preculture

300 mL preculture medium (media composition as described in Example 6) in a glass shake flask were inoculated from a glycerol stock and grown until an optical density (OD550nm) of 1.25 to 2.75 is reached.

### Main culture

The cells were grown in a 7 L (5 L net volume, 3 L batch volume) computer-controlled bioreactor (Sartorius Stedim Biotech AG, Germany) equipped with standard control units. The batch medium is described in Example 6 under "Media Composition". The pH was maintained at a set-point of 6.8 ± 0.2 by addition of 25 % ammonia solution (Merck), the temperature was set to 37 °C ± 0.5 °C and the aeration rate was fixed 5 L per minute. In order to avoid oxygen limitation, the dissolved oxygen level was stabilized above 20 % saturation by stirrer speed and by enrichment with pure oxygen. The content of O₂ and CO₂ in the outlet air was determined by a photoacoustic multi-gas analyzer (Innova). Foaming was suppressed by addition of antifoam suspension (PPG 2000, Dow). For inoculation 30 ml preculture were transferred aseptically to the bioreactor. Feeding was started when the culture entered stationary phase. Fed-Batch regime with an exponential substrate feed was used to provide a constant growth rate of 0.25 h⁻¹ for 8.5 hours and afterwards the feed was changed to a constant mode at the actual feed rate. The feed medium is described in Example 6 under " Media Composition"

### Induction

Induction was performed in a conventional mode by a single pulse directly into the bioreactor. The supplied amount of IPTG was calculated to set a concentration of 1mM IPTG (Isopropyl β-D-1-thiogalactopyranoside) at the end of the process in order to gain a fully induced system.

### Offline Analysis

Optical density (OD)was determined with a Genesis 10 spectrophotometer from Spectronic Instruments, USA, at 550 nm. The OD550nm was measured in a range of 0.2 - 0.6. Above an extinction of 0.6 the samples were appropriately diluted with OD-buffer (20.7 g/L Na₂HPO₄*12H₂O, 5.7 g/L KH₂PO₄ and 11.6 g/L NaCl). Comparable culture broth dilutions as the measured OD-samples were filtered (pore size 0.2 µm) to generate blank values.

Bacterial dry matter (dry cell weight, DCW) was determined by centrifugation of 10 ml of the cell suspension, re-suspension in distilled water followed by centrifugation, and again re-suspension. Then the cell dry weight was determined using a Moisture analyzer (Sartorius Stedim Biotech AG. Germany)

Samples of 35 g culture broth were centrifuged. The supernatant was discarded, residual liquid removed and the weight of the biomass pellet determined.

Glucose in the culture supernatant was measured with the Glucose Analyzer YSI 2700 Select (Yellow Springs).

The content of recombinant protein is determined by reducing SDS Page as follows:
- Sample preparation: BugBuster Extraction Reagent (Novagen-Merck) plus Lysonase Bioprocessing Reagent mix (Novagen-Merck)
- Gel: NuPage 12% Bis Tris, 1.0mm (Invitrogen)
- Sample buffer: NuPAGE LDS Sample Buffer (Invitrogen)
- Reducing agent: 2-Mercaptoethanol (Sigma-Aldrich)
- Running buffer: NuPAGE MES SDS Running Buffer (Invitrogen)
- Detection: SimplyBlue SafeStain (Invitrogen)

### Results

Both fusion proteins, D21N^{pro}37-MCP-1, SEQ.ID. NO: 17 (full length N^{pro} fusion), and D112N^{pro}37-MCP1, SEQ. ID. NO 18 (C-terminal fusion, were expressed as insoluble Inclusion Bodies (IB)(Fig. 6 and 7)

### Summary:

Fig 6 and 7 clearly show, that the titer for MCP-1 (SEQ. ID. NO. C) alone was significantly increased by using the C-terminal _{N}^{pro}-fusion (D112N^{pro}37-MCP-1 (SEQ. ID. NO. 16) instead of the full length N^{pro}-fusion (D21N^{pro}37-MCP-1 (SEQ. ID. NO. 17) for expression. The volumetric (g of MCP-1 per liter of culture broth) as well as the specific (mg of MCP-1 per g of cell dry weigt, DCW) titer were increased 2.4 - fold.

### Example 6: Expression of complementation fragments D21N^{pro}37-6H D107-168, SEQ. ID. NO. 1 (N-terminal N^{pro}), D112N^{pro}37-pep6His, SEQ. ID. NO. 4 (C-terminal fusion), and D21N^{pro}37-pep6His, SEQ. ID. NO 19 (full length N^{pro} fusion) with E. coli in fed batch mode

### Generation of Bacterial Strains and Description of Recombinant Proteins

### Molecular weights:

D112N^{pro}37-pep6His (SEQ. ID. NO. 4): 8.3 kD
D21N^{pro}37-pep6His (SEQ.ID.NO. 19): 18.6 kD
pep6His (SEQ.ID.NO. 20): 1.8 kD:
D21N^{pro}37-6H D107-168 (SEQ.ID.NO. 1) : 10.5 kD

### Sequences:

D21N^{pro}37-pep6His (SEQ. ID: NO. 19) 
MEPVYDTAGR PLFGNPSEVH PQSTLKLPHD RGRGDIRTTL RDLPRKGDCR
SGNHLGPVSG IYIKPGPVYY QDYTGPVYHR APLEFFDETQ FEETTKRIGR
VTGSDGKLYH IYVEVDGEIL LKLAKRGTPR TLKWTRNTTN CPLWVTSCSV
 DKLAAALEHH HHHH
Pep6His (SEQ.ID.NO. 20)
   SV DKLAAALEHH HHHH

**Table 2: List of generated expression stains**

| Protein | Strain | Plasmid | Promoter |
|---|---|---|---|
| D21N^{pro}37-6H D107-168 (SEQ.ID.NO. 1) | BL21 (DE3) | pET30a | T7 |
| D112N^{pro}37-pep6His (SEQ. ID. NO. 4) | BL21 (DE3) | pET30a | T7 |
| Δ21N^{pro}37-pep6HiS (SEQ. ID. NO. 19) | BL21 (DE3) | pET30a | T7 |

Experiments were performed with the B-strain BL21(DE3) provided by Novagen. The designation (DE3) indicates that the hosts were lysogens of λ DE3 prophage, carrying a chromosomal copy of the T7 RNA polymerase gene under control of the lacUV5 promoter, making these strains suitable for protein expression using T7 or T7 lac promoters (Studier and Moffat, 1986; Studier et al., 1990). Expression of target proteins was performed with pET30a plasmid from Novagen (pET System manual, 11^{th} edition).

### Cultivation Mode and Process Analysis

The cells were grown in a 10 L (5 L working volume) computer-controlled bioreactor (MBR; Wetzikon, CH) equipped with standard control units. The pH is maintained at a set-point of 7.0 ± 0.05 by addition of 25 % ammonia solution (ACROS Organics), the temperature was set to 37 °C ± 0.5 °C. In order to avoid oxygen limitation, the dissolved oxygen level was stabilized above 30 % saturation by stirrer speed and aeration rate control. The content of O₂ and CO₂ in the outlet air was determined by a Hartmann and Braun Advanced Optima gas analyzer. Dielectric capacity and conductivity were measured with the Biomass monitor, model 214M (Aber Instruments, Aberystwyth, UK) set. Foaming was suppressed by addition of antifoam suspension (PPG2000, Bussetti, Vienna) with a concentration of 0.5 ml/l feed medium. For inoculation, a deep frozen (-80 °C) working cell bank vial, was thawed and 1 ml (optical density OD₆₀₀= 1) was transferred aseptically to the bioreactor. Feeding is started when the culture, grown to a bacterial dry matter of 7.5 g in 4.0 L batch medium, entered stationary phase. Fed-Batch regime with an exponential substrate feed was used to provide a constant growth rate of 0.2 h⁻¹ during 2 doubling times. The substrate feed was controlled by increasing the pump speed according to the exponential growth algorithm, x=xₒ.e^{µt}, with superimposed feedback control of weight loss in the substrate tank (Cserjan-Puschmann et al., 1999). The feed medium provided sufficient components to yield 129 g of bacterial dry matter.

### Induction

Induction is performed in a conventional mode by a single pulse directly into the bioreactor. The supplied amount of IPTG was calculated to set a concentration of 20 µmol IPTG / g DCW at the end of the process in order to gain a fully induced system.

### Media Composition

The minimal medium used was this example contained 3 g KH₂PO₄ and 6 g K₂HPO₄*3H₂O per liter. These concentrations provide the required buffer capacity and serve as P and K source as well. The other components wee added in relation of gram bacterial dry matter to be produced: sodium citrate (trisodium salt *2H₂O; ACROS organics) 0.25 g, MgSO₄*7H₂O 0.10 g, CaCl₂*2H₂O 0.02 g, trace element solution 50 µl and glucose*H₂O 3 g. To accelerate initial growth of the population, the complex component yeast extract 0.15 g is added to the minimal medium to obtain the batch medium. For the feeding phase 1 L of minimal medium are prepared according to the amount of biological dry matter 129 g to be produced in the feeding phase, whereby P-salts are again added per liter. Trace element solution: prepared in 5 N HCl (g/L): FeSO₄*7H₂O 40.0, MnSO₄*H₂O 10.0, AlCl₃*6H₂O 10.0, CoCl₂ (Fluka) 4.0, ZnSO₄*7H₂O 2.0, Na₂MoO₂*2H₂O 2.0, CuCl₂*2H₂O 1.0, H₃BO₃ 0.50.

### Offline Analysis

Optical density (OD) was measured at 600 nm. Bacterial dry matter (dry cell weight, DCW) was determined by centrifugation of 10 ml of the cell suspension, re-suspension in distilled water followed by centrifugation, and re-suspension for transfer to a pre-weighed beaker, which was then dried at 105 °C for 24 h and re-weighed.

The progress of bacterial growth was determined by calculating the total amount of biomass (DCW).

The quantification of the expressed fusion protein as illustrated in Fig. 8 and 9 was performed with SDS-PAGE by means of a linear regression curve of a reference Therefore, the samples of solubilized IBs were diluted to be within the calibration range and protein content of bands was determined densitometrically by the ImageQuantTL Software.
Cserjan-Puschmann, M.; Kramer, W.; Durrschmid, E.; Striedner, G.; Bayer, K. Metabolic approaches for the optimisation of recombinant fermentation processes. Appl. Microbiol. Biotechnol, 1999, 53, 43-50*.*
Studier, F.W., and Moffatt, B.A. Use of the bacteriophage T7 RNA polymerase to direct selective high-level expression of cloned genes. J. Mol. Biol., 1986, 189, 113-130*.*
Studier, F. W.; Rosenberg, A. L.; Dunn, J. J.; Dubendorff, J. W. Use of T7 RNA polymerase to direct expression of cloned genes. Meth. Enzym., 1990, 185, 60-89*.*

### Results:

Expression systems listed in Table 2 are used to produce the N-terminal N^{pro} fragment and the C-terminal N^{pro}-fusion for the complementation system as well as the full length N^{pro} fusion protein as a reference. Induction is performed as single pulse one doubling past feed start in order to gain a fully induced system.

Both fusion proteins, D112N^{pro}37-pep6His, SEQ.ID.NO. 4 (C-terminal fusion,) and D21N^{pro}37-pep6His, SEQ.ID.NO. 19 (full length Npro fusion), were expressed as insoluble Inclusion Bodies (IB) (Fig. 8 and 9)

### Summary

Fig. 8 and 9 clearly show, that the titer for pep6His (SEQ. ID.NO. E) alone was significantly increased by using the C-terminal N^{pro}-fusion (D112N^{pro}37-pep6His (SEQ. ID. NO. 4) instead of the full length N^{pro}-fusion (D21N^{pro}37-pep6His (SEQ. ID.NO. 19) for expression. The volumetric (g of pep6His per liter of culture broth) as well as the specific (mg of pep6His per g of cell dry weigt, DCW) titer were increased 2.5- and even 2.8-fold respectively.

The N-terminal Npro complementation fragment was expressed with a DCW of 23.8 g/L, a volumetric titer of 0,6 g/L and a specific titer of 0.25 mg / g DCW.

### Example 7:Complementation of complementation fragments D21N^{pro}37-6H D107-168, SEQ.ID.NO. 1 (N-terminal N^{pro}), and D112N^{pro}37-pep6His, SEQ.ID.NO. 4 (C-terminal fusion), and comparison of the cleavage with the full length N^{pro} fusion, D21N^{pro}37-pep6His (SEQ.ID.NO. 19).

The fragments for the complementation system (D21N^{pro}37-6H D107-168, SEQ.ID.NO. 1 and D112N^{pro}37-pep6His, SEQ.ID.NO.4) as well as the full length variant (D21N^{pro}37-pep6His, SEQ.ID.NO.19) used in this Example were produced as described in Example 6.

### Preparation of Inclusion Bodies (IB)

The IBs of the above mentioned proteins were harvested as described previously (Walter et al., 2013). The wet cell paste was harvested using a disk centrifuge (Pathfinder PSC 1-06-177; GEA GEA Westfalia Separator Group, Oelde, Germany) and re-suspended in 50 mM Tris, 50 mM NaCl, and 0.02% Tween at pH 8.0 using an ultra turrax (IKA, Staufen, Germany) to obtain a dry matter concentration of 30 g/L. The slurry was passed twice through a Panda 2K homogenizer (GEA Niro Soavi S.p.A., Italy) at a pressure of 1000 bar. IBs were separated using a disk centrifuge, and the resulting pellet was washed twice with 20 mM Tris, 0.5 M NaCl, and 0.02% Tween at pH 8.0. After centrifugation, the pellet was re-suspended using the ultra turrax and washed once with 0.5 M NaCl. After each washing step, the IBs were separated using the disk centrifuge. The final pellet was re-suspended in water to obtain a 40% IB suspension and stored at -20°C. Prior to the experiments, the IBs were lyophilized and stored at 4°C.
C. Walther, S. Mayer, A. Trefilov, G. Sekot, R. Hahn, A. Jungbauer, A. Durauer, (2013): Prediciton of Inclusion Body Solubilzation From Shaken to Stirred Reactors, Biotechnology & Bioengineering, 111: 84-94

### Solubilization of the Inclusion Bodies.

For solubilization the lyophilized IBs were resuspended 1:5 in water overnight at 4°C. The IB suspension was dissolved at a ratio of 1:10 in the corresponding solubilization buffer. The concentration of buffer ingredients was adjusted taking the dilution factor of IB suspension into account to achieve a resulting concentration of 5M GuHCl (guanidine hydro chloride). Additionally, the GuHCl buffers contained 20 mM Tris and 100 mM NaCl (solubilization buffer "5M GuHCl"). The buffer was adjusted to pH 7.5. Solubilization was performed at low frequency shaking (end-over-end) for 45 minutes. After 45 minutes, solubilization was stopped by centrifugation at 13,200 rpm at 21 °C for 3 min (Centrifuge 5415R, Eppendorf, Germany) and consecutive filtration through 0.22-*µ*m filters (Millipore, Billerica, MA, USA).

### Purification of D21N^{pro}37-6H D107-168, SE.ID. NO. 1 (N-terminal N^{pro}), and D112N^{pro}37-pep6His, SEQ.ID.NO. 4 (C-terminal fusion)

The solubilized IBs of each fragment were purified prior refolding experiments by Ni-NTA Agarose purchased from QIAGEN according to the manual provided. Briefly, solubilized IBs (+ 20 mM Imidazol) were applied on the equilibrated column (solubilization buffer containing 5 M GuHCL + 20 mM Imidazol). After washing with solubilization buffer containing 5 M GuHCL + 20 mM Imidazol the bound fragments of the autoprotease were eluted with solubilization buffer containing 5 M GuHCL + 300 mM Imidazol. The protein concentration was determined by OD_{260/280} measurement. The fraction of the highest protein concentration was used for further processing.

### Refolding, Complementation and Cleavage

Refolding and Cleavage were carried out by rapid dilution of the solubilized IBs into refolding buffer in the ratio 1: 20 keeping either the residual concentration of the chaotrope constant for all experiments and varying the protein concentration or vice versa. As refolding buffers three compositions were used as listed in Table 3.

**Table 3: Buffer compositions used for refoldinq**

| Name | Shortcut | Composition | pH |
|---|---|---|---|
| TRIS buffer | Tris | 50 mM Tris, 30 % Glycerol, 100 mM NaCl; 10 mM MTG | 7.0 |
| Ammonium phosphate buffer | Am-Ph | 50 mM (NH₄)₂HPO4, 30 % Glycerol, 100 mM NaCl, 10 mM MTG | 7.0 |
| Sodium phosphate buffer | Na-Ph | 50 mM NaH₂PO₄ 30 % Glycerol, 100 mM NaCl, 10 mM MTG | 7.0 |

### Determination of the cleavage yield (%)

The analysis of the cleavage yield was carried out as described previously (Walther et al., 2013). The cleavage yield was determined analyzing aliquots of the refolding samples throughout the renaturation process over time by RP-HPLC. Separation of the fusion protein from the cleaved target and autoprotease enables the calculation of the cleaving yield via the increase of the cleaved target / autoprotease compared to the initial overall fusion protein.

RP-HPLC was performed using a TSKgel Super-Octyl column (4.6 x 50/100 mm, 2 µm, 110 Å) (Tosoh Bioscience, Germany). The buffer system consisted of 0.1% (v/v) trifluoroacetic acid (TFA) in water as buffer A and 0.1% (v/v) TFA in acetonitrile as buffer B. Solubilization and refolding samples were injected directly. Elution was performed using different gradients that were optimized for each fusion protein that was analyzed. Detection proceeded at two wavelengths, 214 nm and 280 nm, to distinguish between proteins and buffer components. Calibration curves were established for all fusion proteins to quantify the protein in solution.
C. Walther, S. Mayer, A. Trefilov, G. Sekot, R. Hahn, A. Jungbauer, A. Dürauer, (2013): Prediciton of Inclusion Body Solubilzation From Shaken to Stirred Reactors, Biotechnology & Bioengineering, 111: 84-94

As a reference refolding experiments with the full length fusion protein D21N^{pro}37-pep6His, SEQ.ID.NO. 19, were carried out by rapid dilution of solubilized IB protein into the corresponding refolding buffer. In Table 4 the yield of cleaved target peptide pep6His, SEQ.ID.NO. 20, determined by RP-HPLC after refolding of 24 hours is listed.

**Table 4: Cleavage yield after refolding of the full length fusion protein D21N^{pro}37-pep6His, SEQ.ID.NO. 19, for 24 hours as reference for the complementation system**

| Solubilization buffer | 5M GuHCl | | | |
|---|---|---|---|---|
| Refolding buffer | Na-Ph | | Tris | |
| Refolding concentration [mg/ml] | 0.05 | 0.1 | 0.05 | 0.1 |
| cleavage yield after 24 hours [%] | 42.2 | 42.4 | 50.7 | 52.1 |

Two different strategies were used for refolding, complementation and cleavage for the complementation fragments, D21N^{pro}37-6H D107-168, SEQ.ID.NO. 1 (N-terminal N^{pro}), and D112N^{pro}37-pep6His, SEQ.ID.NO. 4 (C-terminal fusion)

### Method A: Refolding by rapid dilution of both fragments together in a ratio 1:1 into the corresponding refolding buffer

For this approach both fragments were diluted to the 20 fold final refolding concentration with the corresponding solubilization buffer. The fragments were together rapidly diluted 1:20 into the corresponding refolding buffer and aliquots thereof were analysed by RP-HPCL to determine the cleavage yield over time.

**Table 5: Results of Method A: Refolding of complementation fragments D21Npro37-6H D107-186, SEQ.ID.NO. 1, and D112N^{pro}37-pep6His, SEQ.ID.NO. 4, by rapid dilution of both fragments into the corresponding refolding buffer in a concentration ratio of 1:1. The refolding concentration corresponds to the final concentration of each fragment in the refolding buffer. See also Fig. 10.**

| Solubilizatio n buffer | 5 M GuHCl | | | | | | | |
|---|---|---|---|---|---|---|---|---|
| Refolding buffer | Am-Ph | | | Na-Ph | | | Tris | |
| Refolding concentration [mg/mL] | 0.012 5 | 0.02 5 | 0.0 5 | 0.012 5 | 0.02 5 | 0.0 5 | 0.012 5 | 0.02 5 |
| pep6His yield after 144 min [%] | 0 | 2 | 0 | 0 | 2 | 0 | 0 | 2 |
| pep6His yield after 352 min [%] | 0 | 2 | 1 | 3 | 3 | 1 | 3 | 6 |
| pep6His yield after 24 hours [%] | 7 | 23 | 4 | 1 | 26 | 6 | 27 | 33 |

### Method B: Separate refolding of the fragments for defined time span (3, 5 and 24 hours) and subsequent complementation of the fragments for refolding for additional 24 hours

For this approach both fragments were diluted to the 20 fold final refolding concentration with the corresponding solubilization buffer. Each fragment was separately diluted 1:20 into the corresponding refolding buffer and refolded for 3, 5 or 24 hours before aliquots of each fragment were pooled in the ratio 1:1 and refolded for additional 24 hours. Aliquots thereof were analysed by RP-HPCL to determine the cleavage yield over time.

**Table 6: Results of Method B: Refolding of complementation fragments D21Npro37-6H D107-186, SEQ.ID.NO. 1, and D112N^{pro}37-pep6His, SEQ.ID.NO. 4, by rapid dilution of both fragments into the corresponding refolding buffer in a concentration ratio of 1:1 after separate refolding of fragments for 3 hours. The refolding concentration corresponds to the final concentration of each fragment in the refolding buffer.**

| Solubilization buffer | 5 M GuHCl | | | | | |
|---|---|---|---|---|---|---|
| Refolding buffer | Am-Ph | | Na-Ph | | Tris | |
| Refolding concentration [mg/ml] | 0.0125 | 0.025 | 0.0125 | 0.025 | 0.0125 | 0.025 |
| pep6His, SEQ.ID.NO. E, yield 24 hours after pooling [%] | 5 | 11 | 9 | 15 | / | 36 |

**Table 7: Results of Method B: Refolding of complementation fragments D21Npro37-6H D107-186, SEQ.ID.NO. 1, and D112N^{pro}37-pep6His, SEQ.ID.NO. 4, by rapid dilution of both fragments into the corresponding refolding buffer in a concentration ratio of 1:1 after separate refolding of fragments for 5 hours. The refolding concentration corresponds to the final concentration of each fragment in the refolding buffer.**

| Solubilization buffer | 5 M GuHCl | | | | | |
|---|---|---|---|---|---|---|
| Refolding buffer | Am-Ph | | Na-Ph | | Tris | |
| Refolding concentration [mg/ml] | 0.0125 | 0.025 | 0.0125 | 0.025 | 0.01 3 | 0.02 5 |
| pep6His, SEQ.ID.NO. E, yield 24 hours after pooling [%] | 8 | 19 | 15 | 24 | 26 | 34 |

**Table 8: Results of Method B: Refolding of complementation fragments D21Npro37-6H D107-186, SEQ.ID.NO. 1, and D112N^{pro}37-pep6His, SEQ.ID.NO. 4, by rapid dilution of both fragments into the corresponding refolding buffer in a concentration ratio of 1:1 after separate refolding of fragments for 24 hours. The refolding concentration corresponds to the final concentration of each fragment in the refolding buffer.**

| Solubilization buffer | 5 M GuHCl | | | | | |
|---|---|---|---|---|---|---|
| Refolding buffer | Am-Ph | | Na-Ph | | Tris | |
| Refolding concentration [mg/ml] | 0.00625 | 0.0125 | 0.00625 | 0.0125 | 0.00625 | 0.0125 |
| pep6His, SEQ.ID.NO. E, yield 24 hours after pooling [%] | 29 | 4 | 20 | 7 | 47 | 5 |

### Summary:

It was clearly shown, that after complementation of D21Npro37-6H D107-168, SEQ.ID.NO. 1 (N-terminal Npro), and D112Npro37-pep6His, SEQ.ID.NO. 4 (C-terminal fusion), by different modes and under different conditions, the D112Npro37-pep6His, SEQ.ID.NO. 4, was cleaved releasing the pep6His, SEQ.ID.NO. 20 peptide.

The best cleavage yield for the complementation system (36 % after solubilization with 5 M GuHCl, and refolding, complementation and cleavage in Tris-buffer) was 70 % of the cleavage yield of the reference (full length N^{pro} fusion), which had a cleavage yield of 52 %.

### Example 4: Overall process for production of pep6His, SEQ. ID.NO. E by using the full length N^{pro} fusion protein and the C-terminal N^{pro}-fusion protein

The pep6His peptide, SEQ.ID.NO. 20, was produced by using the full length N^{pro} fusion and the C-terminal N^{pro} fusion as descibed in Examples 6 and 7.

### Results:

The volumetric fermentation titer for the sole pep6His, SEQ: ID.NO. 20, was 2.5-fold higher for the complementation system (C-terminal N^{pro} fusion) than for the full length N^{pro} fusion.

The cleavage yield was for the complementation system only 70 % of the cleavage yield of the full length N^{pro} fusion, whereby the cleavage yield for the full length N^{pro} fusion was 52% and for the C-terminal N^{pro} fusion only 36 %.

The overall productivity (g pep6His after cleavage per L culture broth) for the complementation system was 1.7-fold higher than for the full length N^{pro} fusion (Fig.11).

These data show the significant performance of the complementation system compared to the full length N^{pro} system.

### N^{pro} Sequences

D21N^{pro}37-6H D107-168 (N^{pro}505) (SEQ.ID.NO.1): 
MEPVYDTAGR PLFGNPSEVH PQSTLKLPHD RGRGDIRTTL RDLPRKGDCR
SGNHLGPVSG IYIKPGPVYY QDYTGPVYHR APLEFFHHHH HH
6H-D112N^{pro}37-S-Strep (N^{pro}506) (SEQ.ID.NO.2) : 
MHHHHHHETT KRIGRVTGSD GKLYHIYVEV DGEILLKLAK RGTPRTLKWT
RNTTNCPLWV TSCSWSHPQF EK
D112N^{pro}37-S-Strep (N^{pro}507) (SEQ.ID.NO.3) : 
METTKRIGRV TGSDGKLYHI YVEVDGEILL KLAKRGTPRT LKWTRNTTNC
PLWVTSCSWS HPQFEK
D112N^{pro}37-pep6H (N^{pro}584) (SEQ.ID.NO.4) : 
METTKRIGRV TGSDGKLYHI YVEVDGEILL KLAKRGTPRT LKWTRNTTNC
PLWVTSCSVD KLAAALEHHH HHH
D112N^{pro}37-Lysozym-6H (N^{pro}586) (SEQ.ID.NO.5) : 
METTKRIGRV TGSDGKLYHI YVEVDGEILL KLAKRGTPRT LKWTRNTTNC
PLWVTSCMAR VQFKQRESTD AIFVHCSATK PSQNVGVREI RQWHKEQGWL
 
DVGYHFIIKR DGTVEAGRDE MAVGSHAKGY NHNSIGVCLV GGIDDKGKFD
ANFTPAQMQS LRSLLVTLLA KYEGAGLRAH HEVAPKACPS FDLKRWWEKN
ELVTSDRGHH HHHH
Δ21 N^{Pro} (HoBi) (SEQ.ID.NO.13) 
MEPLYDKNGA VLFGEPSDTH PQSTLKLPHP RGEKEVIVGI RDLPRKGDCR
TGNRLGPVSG LFVKPGPVFY QDYSGPVYHR APLEQFKQAP MCEVTKRIGR
VTGSDGNLYH MYVCTDGCIL VKTAKREGQD VLKWVYNVLD SPIWVTSC
N^{Pro} (HoBi) autoprotease moiety (SEQ.ID.NO.21)

MELLNFELLY KTYKQKPAGV QEPLYDKNGA VLFGEPSDTH PQSTLKLPHP
RGEKEVIVGI RDLPRKGDCR TGNRLGPVSG LFVKPGPVFY QDYSGPVYHR
APLEQFKQAP MCEVTKRIGR VTGSDGNLYH MYVCTDGCIL VKTAKREGQD
VLKWVYNVLD SPIWVASC

The SVDKLAAALEHHHHHH motif (SEQ.ID.NO.12) is a model peptide (with His-tag) attached to the autoprotease moiety.

## Claims

1. Method for producing a recombinant protein of interest **characterised by** the following steps:
(a) providing a first part of an N^{pro} autoprotease and providing a second part of an N^{pro} autoprotease, wherein said second part is fused by a peptidic bond to a protein of interest but said second part alone does not exhibit a proteolytic activity, and wherein complementation of said first part with the second part forms an autoproteolytically active N^{pro} autoprotease,
(b) contacting the first part of the N^{pro} autoprotease with the second part of the N^{pro} autoprotease so that an autoproteolytically active N^{pro} autoprotease is formed and the protein of interest fused by the peptidic bond to the second part of the N^{pro} autoprotease is proteolytically cleaved off the second part of the N^{pro} autoprotease at the peptidic bond, and
(c) recovering the protein of interest,
wherein the first part of the N^{pro} autoprotease comprises amino acid 22 to 30 of N^{pro} autoprotease and that the second part of the N^{pro} autoprotease comprises amino acid 113 to 168 of N^{pro} autoprotease.

2. Method according to claim 1, **characterized in that** the first and/or second part of the N^{pro} autoprotease were generated in a recombinant production system, preferably in a prokaryotic host cell, especially in E. coli host cells.

3. Method according to claim 1 or 2, **characterized in that** the first part of the N^{pro} autoprotease comprises amino acid 22 to 75, more preferred amino acid 22 to 112, 22 to 106, 1 to 106, 1 to 75 or 1 to 30, especially amino acid 1 to 112.

4. Method according to any one of claims 1 to 3, **characterized in that** the second part of the N^{pro} autoprotease comprises amino acid 31 to 168 of N^{pro} autoprotease, preferably amino acid 76 to 168, especially amino acid 107 to 168.

5. Method according to any one of claims 1 to 4, **characterized in that** either said first part or said second part of the N^{pro} autoprotease is provided in immobilised form on a solid support and contacted either with said second or said first part of the N^{pro} autoprotease, respectively, in step (b) so that the autoproteolytically active N^{pro} autoprotease is formed on the solid support.

6. Method according to any one of claims 1 to 5, **characterized in that** the first part of the N^{pro} autoprotease and/or the second part of the N^{pro} autoprotease were generated as inclusion bodies.

7. Method according to any one of claims 1 to 6, **characterized in that** the first part of the N^{pro} autoprotease and/or the second part of the N^{pro} autoprotease are also renatured in step (b).

8. N^{pro} autoprotease fragment consisting of amino acid 22 to 30 of Npro autoprotease, preferably amino acid 22 to 75, more preferred amino acid 22 to 112, 22 to 106, 1 to 106, 1 to 75 or 1 to 30, especially amino acid 1 to 112.

9. N^{pro} autoprotease fragment part being fused by a peptidic bond to a protein of interest but not exhibiting a proteolytic activity, consisting of amino acid 31 to 168 of Npro autoprotease, preferably amino acid 76 to 168, especially amino acid 113 to 168 and 107 to 168.

10. Fusion protein comprising a protein of interest and an N^{pro} autoprotease fragment as N-terminal sequence, wherein the N-terminal sequence is selected from amino acids 30 to 168 to 160 to 168 of an N^{pro} sequence.

11. Fusion protein according to claim 10, wherein the N-terminal sequence is selected from amino acids 65 to 168 to 154 to 168 of an N^{pro} molecule, more preferred from amino acid residue 105 to 168 to 140 to 168, especially wherein the N-terminal sequence is selected from 105-168, 106-168, 107-168, 108-168, 109-168, 110-168, 111-168, 112-168, 113-168, 114-168, 115-168, 116-168, 117-168, 118-168, 119-168, 120-168, 121-168, 122-168, 123-168, 124-168, 125-168, 126-168, 127-168, 128-168, 129-168, 130-168, 131-168, 132-168, 133-168, 134-168, 135-168, 136-168, 137-168, 138-168, 139-168, 140-168, 141-168, 142-168, 143-168, 144-168, 145-168, 146-168, 147-168, 148-168, 149-168, 150-168, 151-168, 152-168, 153-168, 154-168, 155-168, 156-168, 157-168, 158-168 159-168, or 160-168 of an N^{pro} sequence.

12. Expression vector encoding for an N^{pro} autoprotease fragment or fragment part or fusion protein according to any one of claims 8 to 11.

13. Host cell, preferably a prokaryotic host cell, especially an E. coli host cell, containing an expression vector according to claim 12.

14. Solid support comprising an N-terminal part (first part) of an N^{pro} autoprotease, wherein complementation of said first part with the C-terminal part (second part) of the N^{pro} autoprotease forms an autoproteolytically active N^{pro} autoprotease.

15. Method for production of a fusion protein according to claim 10 or 11 wherein the fusion polypeptide is recombinantly expressed and purified.

## Patentansprüche

1. Verfahren zur Herstellung eines rekombinanten Proteins von Interesse, **gekennzeichnet durch** die folgenden Schritte:
(a) Bereitstellen eines ersten Teils einer N^{pro}-Autoprotease und Bereitstellen eines zweiten Teils einer N^{pro}-Autoprotease, wobei der zweite Teil durch eine peptidische Bindung an ein Protein von Interesse fusioniert ist, wobei aber der zweite Teil alleine keine proteolytische Aktivität aufweist, und wobei die Komplementation des ersten Teils mit dem zweiten Teil eine autoproteolytisch aktive N^{pro}-Autoprotease bildet,
(b) Inkontaktbringen des ersten Teils der N^{pro}-Autoprotease mit dem zweiten Teil der N^{pro}-Autoprotease, so dass eine autoproteolytisch aktive N^{pro}-Autoprotease gebildet wird und das durch die peptidische Bindung an den zweiten Teil der N^{pro}-Autoprotease fusionierte Protein von Interesse vom zweiten Teil der N^{pro}-Autoprotease an der peptidischen Bindung proteolytisch abgespalten wird, und
(c) Rückgewinnen des Proteins von Interesse,
wobei der erste Teil der N^{pro}-Autoprotease Aminosäuren 22 bis 30 der N^{pro}-Autoprotease umfasst und der zweite Teil der N^{pro}-Autoprotease Aminosäuren 113 bis 168 der N^{pro}-Autoprotease umfasst.

2. Verfahren gemäß Anspruch 1, **dadurch gekennzeichnet, dass** der erste und/oder zweite Teil der N^{pro}-Autoprotease in einem rekombinanten Produktionssystem, vorzugsweise in einer prokaryotischen Wirtszelle, insbesondere in einer E. coli-Wirtszelle, generiert wurden.

3. Verfahren gemäß Anspruch 1 oder 2, **dadurch gekennzeichnet, dass** der erste Teil der N^{pro}-Autoprotease Aminosäuren 22 bis 75, mehr bevorzugt Aminosäuren 22 bis 112, 22 bis 106, 1 bis 106, 1 bis 75 oder 1 bis 30, insbesondere Aminosäuren 1 bis 112, umfasst.

4. Verfahren gemäß einem der Ansprüche 1 bis 3, **dadurch gekennzeichnet, dass** der zweite Teil der N^{pro}-Autoprotease Aminosäuren 31 bis 168 der N^{pro}-Autoprotease, vorzugsweise Aminosäuren 76 bis 168, insbesondere Aminosäuren 107 bis 168, umfasst.

5. Verfahren gemäß einem der Ansprüche 1 bis 4, **dadurch gekennzeichnet, dass** entweder der erste Teil oder der zweite Teil der N^{pro}-Autoprotease in immobilisierter Form an einem festen Träger bereitgestellt wird und entweder mit dem zweiten beziehungsweise mit dem ersten Teil der N^{pro}-Autoprotease in Schritt (b) in Kontakt gebracht wird, so dass die autoproteolytisch aktive N^{pro}-Autoprotease an dem festen Träger gebildet wird.

6. Verfahren gemäß einem der Ansprüche 1 bis 5, **dadurch gekennzeichnet, dass** der erste Teil der N^{pro}-Autoprotease und/oder der zweite Teil der N^{pro}-Autoprotease als Einschlusskörperchen generiert wurden.

7. Verfahren gemäß einem der Ansprüche 1 bis 6, **dadurch gekennzeichnet, dass** der erste Teil der N^{pro}-Autoprotease und/oder der zweite Teil der N^{pro}-Autoprotease in Schritt (b) außerdem renaturiert werden.

8. N^{pro}-Autoprotease-Fragment, bestehend aus Aminosäuren 22 bis 30 der N^{pro}-Autoprotease, vorzugsweise Aminosäuren 22 bis 75, mehr bevorzugt Aminosäuren 22 bis 112, 22 bis 106, 1 bis 106, 1 bis 75 oder 1 bis 30, insbesondere Aminosäuren 1 bis 112.

9. N^{pro}-Autoprotease-Fragmentteil, der durch eine peptidische Bindung an ein Protein von Interesse fusioniert ist, doch keine proteolytische Aktivität aufweist, bestehend aus Aminosäuren 31 bis 168 der N^{pro}-Autoprotease, vorzugsweise Aminosäuren 76 bis 168, insbesondere Aminosäuren 113 bis 168 und 107 bis 168.

10. Fusionsprotein, umfassend ein Protein von Interesse und ein N^{pro}-Autoprotease-Fragment als N-terminale Sequenz, wobei die N-terminale Sequenz ausgewählt ist aus Aminosäuren 30 bis 168 hin bis 160 bis 168 einer N^{pro}-Sequenz.

11. Fusionsprotein gemäß Anspruch 10, wobei die N-terminale Sequenz ausgewählt ist aus Aminosäuren 65 bis 168 hin bis 154 bis 168 eines N^{pro}-Moleküls, mehr bevorzugt aus Aminosäureresten 105 bis 168 hin bis 140 bis 168, insbesondere wobei die N-terminale Sequenz ausgewählt ist aus 105-168, 106-168, 107-168, 108-168, 109-168, 110-168, 111-168, 112-168, 113-168, 114-168, 115-168, 116-168, 117-168, 118-168, 119-168, 120-168, 121-168, 122-168, 123-168, 124-168, 125-168, 126-168, 127-168, 128-168, 129-168, 130-168, 131-168, 132-168, 133-168, 134-168, 135-168, 136-168, 137-168, 138-168, 139-168, 140-168, 141-168, 142-168, 143-168, 144-168, 145-168, 146-168, 147-168, 148-168, 149-168, 150-168, 151-168, 152-168, 153-168, 154-168, 155-168, 156-168, 157-168, 158-168, 159-168 oder 160-168 einer N^{pro}-Sequenz.

12. Expressionsvektor, der für ein N^{pro}-Autoprotease-Fragment oder -Fragmentteil oder -Fusionsprotein gemäß einem der Ansprüche 8 bis 11 codiert.

13. Wirtszelle, vorzugsweise eine prokaryotische Wirtszelle, insbesondere eine E. coli-Wirtszelle, enthaltend einen Expressionsvektor gemäß Anspruch 12.

14. Fester Träger, umfassend einen N-terminalen Teil (erster Teil) einer N^{pro}-Autoprotease, wobei die Komplementation des ersten Teils mit dem C-terminalen Teil (zweiter Teil) der N^{pro}-Autoprotease eine autoproteolytisch aktive N^{pro}-Autoprotease bildet.

15. Verfahren zur Herstellung eines Fusionsproteins gemäß Anspruch 10 oder 11, wobei das Fusionspolypeptid rekombinant exprimiert und gereinigt wird.

## Revendications

1. Procédé de production d'une protéine recombinée d'intérêt **caractérisé par** les étapes suivantes :
(a) utilisation d'une première partie d'une autoprotéase N^{pro} et utilisation d'une seconde partie d'une autoprotéase N^{pro}, où ladite seconde partie est fusionnée par une liaison peptidique à une protéine d'intérêt mais ladite seconde partie seule ne manifeste pas d'activité protéolytique, et où la complémentation de ladite première partie avec la seconde partie forme une autoprotéase N^{pro} à activité autoprotéolytique,
(b) mise en contact de la première partie de l'autoprotéase N^{pro} avec la seconde partie de l'autoprotéase N^{pro} de façon qu'une autoprotéase N^{pro} à activité autoprotéolytique soit formée et que la protéine d'intérêt fusionnée par la liaison peptidique à la seconde partie de l'autoprotéase N^{pro} soit séparée par clivage protéolytique de la seconde partie de l'autoprotéase N^{pro} au niveau de la liaison peptidique, et
(c) récupération de la protéine d'intérêt,
dans lequel la première partie de l'autoprotéase N^{pro} comprend les acides aminés 22 à 30 de l'autoprotéase N^{pro} et la seconde partie de l'autoprotéase N^{pro} comprend les acides aminés 113 à 168 de l'autoprotéase N^{pro}.

2. Procédé selon la revendication 1, **caractérisé en ce que** la première et/ou la seconde partie de l'autoprotéase N^{pro} a et/ou ont été générée (s) dans un système de production recombinant, de préférence chez une cellule hôte procaryote, notamment chez des cellules hôtes de type E. coli.

3. Procédé selon la revendication 1 ou 2, **caractérisé en ce que** la première partie de l'autoprotéase N^{pro} comprend les acides aminés 22 à 75, plus préférablement les acides aminés 22 à 112, 22 à 106, 1 à 106, 1 à 75, ou 1 à 30, notamment les acides aminés 1 à 112.

4. Procédé selon l'une quelconque des revendications 1 à 3, **caractérisé en ce que** la seconde partie de l'autoprotéase N^{pro} comprend les acides aminés 31 à 168 de l'autoprotéase N^{pro}, de préférence les acides aminés 76 à 168, notamment les acides aminés 107 à 168.

5. Procédé selon l'une quelconque des revendications 1 à 4, **caractérisé en ce que**, soit ladite première partie, soit ladite seconde partie de l'autoprotéase N^{pro} est fournie sous une forme immobilisée sur un support solide et mise en contact soit avec ladite seconde, soit avec ladite première partie de l'autoprotéase N^{pro}, respectivement, à l'étape (b) de façon que l'autoprotéase N^{pro} à activité autoprotéolytique soit formée sur le support solide.

6. Procédé selon l'une quelconque des revendications 1 à 5, **caractérisé en ce que** la première partie de l'autoprotéase N^{pro} et/ou la seconde partie de l'autoprotéase N^{pro} a et/ou ont été générée (s) sous la forme de corps d'inclusion.

7. Procédé selon l'une quelconque des revendications 1 à 6, **caractérisé en ce que** la première partie de l'autoprotéase N^{pro} et/ou la seconde partie de l'autoprotéase N^{pro} est et/ou sont également renaturée (s) à l'étape (b).

8. Fragment d'autoprotéase N^{pro} constitué par les acides aminés 22 à 30 de l'autoprotéase N^{pro}, de préférence les acides aminés 22 à 75, plus préférablement les acides aminés 22 à 112, 22 à 106, 1 à 106, 1 à 75 ou 1 à 30, notamment les acides aminés 1 à 112.

9. Partie de fragment d'autoprotéase N^{pro} fusionnée par une liaison peptidique à une protéine d'intérêt mais ne manifestant pas d'activité protéolytique, constituée par les acides aminés 31 à 168 de l'autoprotéase N^{pro}, de préférence les acides aminés 76 à 168, notamment les acides aminés 113 à 168 et 107 à 168.

10. Protéine de fusion comprenant une protéine d'intérêt et un fragment d'autoprotéase N^{pro} à titre de séquence N-terminale, dans laquelle la séquence N-terminale est choisie parmi les acides aminés 30 à 168 à 160 à 168 d'une séquence N^{pro}_{.}

11. Protéine de fusion selon la revendication 10, dans laquelle la séquence N-terminale est choisie parmi les acides aminés 65 à 168 à 154 à 168 d'une molécule N^{pro}, plus préférablement parmi les résidus acides aminés 105 à 168 à 140 à 168, notamment dans laquelle la séquence N-terminale est choisie parmi 105-168, 106-168, 107-168, 108-168, 109-168, 110-168, 111-168, 112-168, 113-168, 114-168, 115-168, 116-168, 117-168, 118-168, 119-168, 120-168, 121-168, 122-168, 123-168, 124-168, 125-168, 126-168, 127-168, 128-168, 129-168, 130-168, 131-168, 132-168, 133-168, 134-168, 135-168, 136-168, 137-168, 138-168, 139-168, 140-168, 141-168, 142-168, 143-168, 144-168, 145-168, 146-168, 147-168, 148-168, 149-168, 150-168, 151-168, 152-168, 153-168, 154-168, 155-168, 156-168, 157-168, 158-168, 159-168, ou 160-168 d'une séquence N^{pro}.

12. Vecteur d'expression codant pour un fragment ou une partie de fragment d'autoprotéase N^{pro} ou pour une protéine de fusion selon l'une quelconque des revendications 8 à 11.

13. Cellule hôte, de préférence cellule hôte procaryote, notamment cellule hôte de type E. coli, contenant un vecteur d'expression selon la revendication 12.

14. Support solide comprenant la partie N-terminale (première partie) d'une autoprotéase N^{pro}, où la complémentation de ladite première partie avec la partie C-terminale (seconde partie) de l'autoprotéase N^{pro} forme une autoprotéase N^{pro} à activité autoprotéolytique.

15. Procédé de production d'une protéine de fusion selon la revendication 10 ou 11 dans lequel le polypeptide de fusion est exprimé par recombinaison et purifié.
